# EUROPÄISCHE PATENTSCHRIFT

(11) **EP 1 626 952 B1**
(45) Veröffentlichungstag und Bekanntmachung des Hinweises auf die Patenterteilung: **04.06.2014**
(21) Anmeldenummer: 02799716.2
(22) Anmeldetag: 21.12.2002
(51) Int. Cl.: C07F 9/6553, C07D 339/00, C07D 339/04, C07D 339/08, C07C 323/16, C07F 9/24

(54) **MULTIFUNKTIONALES REAGENZ ZUR SYNTHESE VON THIOLMODIFIZIERTEN OLIGOMEREN**
MULTIFUNCTIONAL REAGENT FOR THE SYNTHESIS OF THIOL MODIFIED OLIGOMERS
REACTIF MULTIFONCTIONNEL UTILISE POUR SYNTHETISER DES OLIGOMERES A MODIFICATION THIOL

(30) Priorität: 22.12.2001 DE 10163836
(43) Veröffentlichungstag der Anmeldung: 22.02.2006
(73) Patentinhaber: FRIZ Biochem Gesellschaft für Bioanalytik mbH, 82061 Neuried (DE)
(72) Erfinder: HARTWICH, Gerhard, 80639 München (DE); FRISCHMANN, Peter, 82145 München (DE); FERRER, Elisenda, 81825 München (DE)
(74) Vertreter: Graf Glück Kritzenberger
(86) Internationale Anmeldenummer: PCT/DE2002/004699
(87) Internationale Veröffentlichungsnummer: WO 2003/055852

(56) Entgegenhaltungen:
- DATABASE CA [Online] CHEMICAL ABSTRACTS SERVICE, COLUMBUS, OHIO, US; SCHEPINOV, M. S. ET AL: "Selectively cleavable synthetic oligodeoxyribonucleotides for the reversible immobilization of DNA" retrieved from STN Database accession no. 123:199284 CA XP002257908 & BIOORGANICHESKAYA KHIMIYA (1994), 20(8-9), 955-66 ,
- DATABASE CA [Online] CHEMICAL ABSTRACTS SERVICE, COLUMBUS, OHIO, US; KATO, HISATOYO ET AL: "preparation of glycerin derivatives for DNA probes" retrieved from STN Database accession no. 121:134692 CA XP002257909 & JP 06 072990 A (TOA GOSEI CHEM IND, JAPAN) 15. März 1994 (1994-03-15)

## Beschreibung

### Technisches Gebiet

Die Erfindung betrifft ein multifunktionales Reagenz zur Synthese von thiolmodifizierten Oligomeren.

### Stand der Technik

Nukleinsäuren können chemisch oder enzymatisch synthetisiert werden. Je nach Art der eingesetzten Nukleotidbausteine und der Reaktionsschritte zur Anknüpfung an das in der Sequenz benachbarte Nukleotid unterscheidet man verschiedene Verfahren: Phosphodiester-, Phosphotriester- und Phosphoramidit-Verfahren (Gait, M.J. et al., Oligonucleotide Synthesis: A Practical Approach, IRL Press Oxford, 1984; Protocols for Oligonucleotides and Analogs, Agrawal, S., Humana Press, New Jersey, 1993). Beim Phosphoramidit-Verfahren werden keine Phosphorsäurederivate, sondern Derivate der phosphorigen Säure, sogenannte Phosphoramidite eingesetzt.

Das Phosphoramidit-Verfahren kann als Festphasenverfahren durchgeführt werden. Dabei ist die wachsende Nukleotidsequenz an einem polymeren Träger gebunden. Die Abtrennung der überschüssigen Synthesereagenzien und -bausteine sowie die Reinigung der Oligonukleotidsequenz wird dadurch stark vereinfacht. Kommerziell erhältliche Nukleinsäure-Syntheseautomaten arbeiten nach diesem Prinzip.

Nukleinsäuren mit bekannter Nukleotidsequenz finden insbesondere Anwendung zum spezifischen Nachweis von DNA in biologischen Proben. In solchen Nachweisverfahren wird die Eigenschaft ausgenutzt, dass einzelsträngige Nukleotidsequenzen mit ihrem komplementären Gegenstrang einen Doppelstrang ausbilden können. Der Vorgang der Doppelstrangbildung wird Hybridisierung genannt (Nucleic Acids in Chemistry and Biology, Blackburn, M.G. und Gait, J.M., Oxford University Press).

Die Bildung eines Doppelstranges kann nachgewiesen werden, wenn zur Hybridisierung mit der einzelsträngigen Nukleinsäure eine modifizierte einzelsträngige komplementäre Nukleinsäure eingesetzt wird oder die einzelsträngige Nukleinsäure selbst eine Modifikation trägt. Modifizierungen können u. a. Fluorophore, Radioisotope oder Elektrolabel sein.

Die Verwendung von markierten Targets zum Nachweis von Hybridisierungsereignissen beinhaltet jedoch einige Nachteile. Zum einen muss die Markierung vor der eigentlichen Messung erfolgen, was einen zusätzlichen Syntheseschritt und damit zusätzlichen Arbeitsaufwand bedeutet. Zudem ist es schwierig, eine homogene Markierung des Probenmaterials zu gewährleisten. Außerdem sind stringente Waschbedingungen notwendig, um im Anschluss an eine Hybridisierung nicht oder unspezifisch gebundenes Material zu entfernen.

Oligonukleotide und im Allgemeinen Polymere können auf Oberflächen durch bekannte Methoden wie z.B. durch nicht kovalente Adsorption oder durch kovalente Kupplungen auf einer Oberfläche immobilisiert werden (WO 00/42217; US 6,312,906 Bioorganicheskaya Khimiya (1994), 20(8-9), 955-966). Besonders vorteilhafte Verfahren um Oligonukleotide auf einer SiO₂-Oberfläche (Glas) zu immobilisieren, basieren auf der gut etablierten Silicon-Chemie (Parkam et al., Biochem. Biophys. Res. Commun., 1:1-6, 1978; Lund et al., Nucl. Acids Res. 16 :10861-10880, 1988). So können z.B. epoxid-modifizierte SiO₂-Oberflächen mit aminofunktionalisierten Oligonukleotiden belegt werden.

Die Chemisorption an Gold wurde erst nach 1983 näher untersucht. Nuzzo und Allara (J. Am. Chem. Soc. 105, 4481, 1983) entdeckten, dass Thiole und Disulfide auf Gold in geordneten Monolayern adsorbiert werden. Die entstehende kovalente Bindung zwischen Gold und Schwefel hat eine Bindungsenergie von 30-40 kcal/mol. Bain et al. (J. Am. Chem. Soc. 111, 321, 1989; J. Am. Chem. Soc. 111, 7155, 1989) beschrieben die Bindungsverhältnisse zwischen Organoschwefelverbindungen und Gold. Die starke, koordinative Gold-Schwefel-Bindung treibt die spontane Anlagerung zu Monolayern voran. Bain et al. argumentieren, dass die Bildung dieser Monolayer durch viele Faktoren (wie z.B. Temperatur, Lösungsmittel, Konzentration und Kettenlänge des Adsorbats, Salzkonzentration) beeinflusst wird. Es gibt zwei Phasen der Adsorption. Die Ausbildung einer ersten Monolayerschicht, die etwa 80-90% der Oberfläche belegt, ist in einigen Minuten abgeschlossen. Die restliche Fläche wird in einem Prozess, der sich über mehrere Stunden hinzieht, belegt. Dabei spielen wahrscheinlich Verdrängungsprozesse auf der Oberfläche (z.B. Lösungsmittel) und laterale Diffusionen eine Rolle. Diese Experimente stellen die Grundlagen für die Anbindung von thiolmodifizierten Oligonukleotiden dar.

An eine Gold-Oberfläche über nur einen Thiol-Anker angebundene Oligonukleotide, vereinfacht ausgedrückt durch den Begriff "Au-S-Oligonukleotid", sind bei mechanischer Beanspruchung (wie z.B. bei Waschschritten) instabil. Die Stabilität des Oligonukleotids auf der Oberfäche wird durch mehrfach ausgebildete Au-S-Bindungen erhöht. Eine solche sehr stabile Anbindung der Oligonukleotide bringt erhebliche Vorteile für die DNA-Chip-Technologie mit sich.

Eine Variante zur Anbindung von DNA an Gold- oder Platin-Oberflächen stellt das von Whitesides und Mitarbeitern entwickelte Verfahren (Lee et al., Pure & Appl. Chem. 63, 821, 1991) zur Generierung von Thiol-Monoschichten auf Gold-Oberflächen dar. Dabei wird die freie Thiol-Gruppe auf einer mit einem Dithiol (z.B. 1,10-Decandithiol) vorbelegten Metalloberfläche mit einem bromacetylmodifizierten Oligonukleotid umgesetzt.

Zur Darstellung von thiolmodifizierten Oligonukleotiden können schwefelhaltige Phosphoramidite oder polymere Trägermaterialien eingesetzt werden. Beispiele von Verbindungen, die zur Ankopplung einer Disulfid-Einheit dienen, sind das Phosphoramidit DMT-O-(CH₂)₆-S-S-(CH₂)₆-O-P(OCE)(NiPr₂) oder Verbindungen mit der allgemeinen Strukturformel R¹-S-S-R²-O-P(OCE)(NiPr₂) (siehe EP 523 978). Eine andere Möglichkeit einen Thiol-Anker an ein Oligonukleotid anzukoppeln, ist die Verwendung des Phosphoramidits MMT-S-(CH₂)₆-O-P(OCE)(NiPr₂). Der Nachteil bei Verwendung dieses Phosphoramidits ist die aufwändige Abspaltung der MMT Gruppe mit AgNO₃.

Bekannt sind weiterhin auch zwei Thiol-Trägermaterialen mit C-3 und C-6 Spacer für die Oligonukleotid-Synthese (Glen Research).

Trotz des beschriebenen Standes der Technik besteht weiterhin ein Bedarf an multifunktionellen thiolhaltigen Monomeren, die einen polyfunktionalen Thiol-Anker ausbilden, mit dessen Hilfe eine stabile Anbindung von Molekülen oder Polymeren auf Oberflächen oder polymeren Trägern möglich ist.

### Darstellung der Erfindung

Aufgabe der vorliegenden Erfindung ist es daher thiolhaltige Monomere zur Verfügung zu stellen, die die Darstellung polyfunktionaler Thiol-Verbindungen erlauben.

Diese Aufgabe wird erfindungsgemäß durch die Verbindungen gemäß unabhängigem Anspruch 1 gelöst. Weitere vorteilhafte Details, Aspekte und Ausgestaltungen der vorliegenden Erfindung ergeben sich aus den abhängigen Ansprüchen, der Beschreibung, den Figuren und den Beispielen.

Im Rahmen der vorliegenden Erfindung werden die folgenden Abkürzungen und Begriffe benutzt:
- A:: Adenin
- ACN:: Acetonitril
- Base:: A, G, T, C oder U
- C:: Cytosin
- DC:: Dünnschichtchromatographie
- DMT:: 4,4'-Dimethoxytrityl
- DNA:: Desoxyribonukleinsäure
- E^{∼}:: Wechselspannung
- EI:: Electrospray Ionisation
- EtOAc:: Ethylacetat
- Et₃N:: Triethylamin
- f :: Wechselspannungsfrequenz
- Fmoc:: 9-Fluorenylmethoxycarbonyl
- G:: Guanin
- HPLC :: Hochdruckflüssigkeitschromatographie
- iPr:: Isopropyl
- NMR :: Kernmagnetische Resonanz
- M:: Masse
- MsCl:: Mesylchlorid oder Methansulfonylchlorid
- MeOH:: Methanol
- MS:: Massenspektrometrie
- mV:: Millivolt
- C_{q}:: quartäre Kohlenstoffe
- Cₐᵣₒₘ:: aromatische Kohlenstoffe
- Hₐᵣₒₘ:: aromatische Wasserstoffe
- OD₂₆₀:: Optische Dichte (bei 260 nm)
- OCE:: Cyanoethoxy
- Oligomer:: Äquivalent zu Nukleinsäure-Oligomer.
- Oligonukleotid:: Ein DNA-, PNA- oder RNA-Fragment nicht näher spezifizierter Basenlänge.
- PNA:: Peptidnukleinsäure (-NH-(CH₂)₂-N(COCH₂-Base)-CH₂CO; synthetische DNA oder RNA, bei der die Zucker-Phosphat-Einheit durch eine Aminosäure ersetzt ist. PNA kann mit DNA oder RNA hybridisiert werden).
- RNA:: Ribonukleinsäure
- R_{f}:: Retention bei der DC relativ zur Laufmittelfront
- rms:: root mean square
- RP:: Reverse-Phase
- s:: Singulett
- SPR:: Oberflächenplasmonen-Resonanz-Spektroskopie
- T:: Thymin
- U:: Uracil
- v:: Vorschubgeschwindigkeit

Sämtliche Strukturformeln sind so zu verstehen, dass auch die entsprechenden chiralen Enantiomere umfasst sind.

Die vorliegenden Erfindung umfasst

Verbindungen der Formel 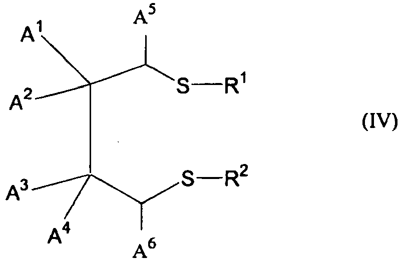 wobei A¹, A³, A⁵ und A⁶ gleich H sind, A⁴ gleich Y² ist, A² ein Alkylrest mit 1-22 C-Atomen, ein Heteroalkylrest mit 1-22 C-Atomen, ein Cycloalkylrest mit 1-22 C-Atomen ist und Schutzgruppe-Y¹ umfasst, oder A² gleich Schutzgruppe-Y¹ ist, R¹ und R² gleiche oder verschiedene H oder Schwefel-Schutzgruppen sind, wobei die beiden S-Atome auch eine Disulfid-Brücke ausbilden können und in diesem Fall R¹, R² nicht vorhanden sind, Schutzgruppe-Y¹ gleich Schutzgruppe-S, Schutzgruppe-NH, Schutzgruppe-NR⁴, Schutzgruppe-O, Schutzgruppe-S-S, Schutzgruppe-OOC ist, Schutzgruppe gleich 9-Fluorenylmethoxycarbonyl oder Dimethoxytrityl ist,
Y² ist -OH, -NH₂, -NHR³, -NR³R⁴, -COOH, -COCI, -COOCO-R⁶, -CONH₂, -CONHR³, -COOR³, -SO₃H, -SO₃Cl, -SH, -S-SR³, -CHO, -COR³, -C₂H₃O, Halogen, -N₃, -NH-NH₂, -NCO, -NCS, wobei R³ Alkyl, Heteroalkyl, Aryl, Cycloalkyl oder eine Schutzgruppe ist, wobei R³ in den Gruppen Y¹ und Y² gleich oder verschieden vorliegen kann, R⁴ eine Schutzgruppe ist, wobei R⁴ in den Gruppen Y¹ und Y² gleich oder verschieden vorliegen kann, und wobei R⁴ und R³ gleich oder verschieden sein können, R⁵ Alkyl, Aryl, Cycloalkyl ist, wobei R⁵ in den Gruppen Y¹ und Y² gleich oder verschieden vorliegen kann, und R⁸ Alkyl, Heteroalkyl, Aryl oder Cycloalkyl ist, wobei R⁶ in den Gruppen Y¹ und Y² gleich oder verschieden vorliegen kann, wobei Y² auch eine Gruppe der Formel (II) oder (III) sein kann, 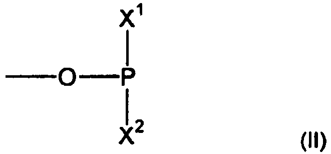 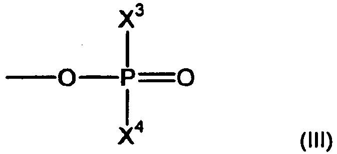 wobei X¹ ein Halogen oder ein substituiertes Amin ist, X² ein Alkyl-, Alkoxy-, Aryloxy-Rest oder ein Cyanoderivat eines Alkyl-, Alkoxy-, Aryloxy-Restes ist, X³ ein Halogen, eine Aminofunktion oder Sauerstoff ist und X⁴ ein Alkyl-, Alkoxy-, Aryloxy-Rest ist oder X⁴ gleich H ist für den Fall, dass X³ = Sauerstoff ist.

Bei den erfindungsgemäßen Verbindungen handelt es sich also um Substanzen mit mindestens vier funktionellen Gruppen, von denen zwei Thiole (R¹, R² = H), Thioether oder Disulfide sind, wobei die beiden Schwefelatome auch mit sich selbst eine Disulfidbrücke ausbilden können und dabei die Reste R¹, R² entfallen. Y¹ ist eine funktionelle Gruppe mit einer Schutzgruppe. Y² ist eine funktionelle Gruppe, die u.a. zur Aktivierung der erfindungsgemäßen Verbindungen für chemische Reaktionen dient. Bei diesen chemischen Reaktionen handelt es sich z.B. um eine Polymerisierung oder um die Anbindung an ein polymeres Trägermaterial. Dabei kann Y² bereits eine aktivierte funktionelle Gruppe oder eine zu aktivierende funktionelle Gruppe darstellen.

Die erfindungsgemäßen Verbindungen können für den gezielten und definierten Aufbau von Oligomeren bzw. Polymeren mit einer exakt definierten Anzahl von Schwefelatomen verwendet werden. Dazu ist die selektive Abspaltung einer Schutzgruppe eine notwendige Bedingung. Die chemische Umsetzung der Monomere darf die Integrität der Schutzgruppe nicht beeinflussen. Die vorhandenen Reste R¹, R² am Schwefel müssen während der Polymerisation, also während der Aktivierung der funktionellen Gruppe und der Abspaltung der Schutzgruppe, chemisch stabil bleiben, d.h. die Schutzgruppe an Y¹ muss orthogonal bzw. selektiv zu den Resten R¹, R² am Schwefel abspaltbar sein.

Sind die Reste R¹ und R² nicht vorhanden und bilden die beiden Schwefelatome aus diesem Grund eine Disulfidbrücke aus, so stellt die Tatsache einen besonderen Vorteil dar, dass die Disulfideinheiten nicht im Rückgrat der Polymere angeordnet sind, wodurch ein Spalten der Disulfidbrücke/n keinen Abbau der Polymere verursacht.

Schutzgruppen sind 9-Fluorenylmethoxycarbonyl oder Dimethoxytrityl-.

R¹ und R² sind gleiche oder verschiedene Schwefel-Schutzgruppen, die u.a. Benzylderivate, Triphenylmethylderivate, substituierte Methylderivate, Benzoyl-, Trifluoracetyl- oder t-Butoxycarbonylgruppen sein können. Weiterhin können die Thiole als Disulfide wie z.B. S-Ethyldisulfide, S-Phenyldisulfide oder als Thiocarbamate geschützt sein. Weitere Schwefel-Schutzgruppen kann man in Greene, T. W., Protective Groups in Organic Synthesis, Wiley-Interscience, 1999 finden.

Sämtliche erfindungsgemäßen Verbindungen können als monomere Einheit in Oligomere eingebaut werden.

Die erfindungsgemäßen Verbindungen können unabhängig von der Position an Nukleotid-Oligomeren, Peptid-Oligomeren oder anderen Molekülen ein- oder mehrfach angebracht werden. Dadurch wird z.B. die Anbindung von beliebigen Molekülen und auch Polymeren über einen Polythiol-Anker an Oberflächen oder an polymere Träger ermöglicht. Durch Aktivierung des polyfunktionalen Thiol-Ankers entsteht eine mehrfache Anbindung des Moleküls an Oberflächen (z.B. an eine Gold-Oberfläche) oder an polymere Träger. Dazu können speziell voraktivierte Oberflächen (z.B. mit Aldehyd oder Maleinimid aktivierte Oberflächen) oder spezielle polymere Verbindungen wie z.B. Peptide, Proteine, PNA, RNA, LNA (Locked Nucleic Acids) verwendet werden, die gegenüber Thiolen reaktive Gruppen enthalten. Die an Molekülen, insbesondere an Polymeren angebrachten erfindungsgemäßen Verbindungen können auch zum Anbinden von Markierungen (Labeln) oder anderen Funktionalitäten an die Moleküle, insbesondere an die Polymere verwendet werden.

Ein Vorteil der erfindungsgemäßen Verbindungen ist die Möglichkeit, mehrfache SH-Gruppen in ein Oligomer einzuführen und dadurch höhere Stabilität bei Immobilisierung des Oligomers auf einer Oberfläche zu bekommen. Einen weiteren Vorteil stellt die Möglichkeit dar, durch mehrfache SH-Gruppen an einem Polymer verschiedene Moleküle wie z.B. Polymere, Peptide, Proteine oder Oligonukleotide anzukoppeln.

Y² kann auch direkt oder über einen Linker an einem festen Trägermaterial angekoppelt sein, wie z.B. CPG (Controlled Pore Glass), Microbeads, Polymere (z.B. Polystyrene) oder Membranen. Um Nukleinsäuren synthetisch herzustellen werden die erfindungsgemäßen Verbindungen normalerweise mittels eines Aminoalkyrestes (LCAA = Long Chain Alkyl Amine) an einen festen Träger angebunden.

Die im Rahmen der vorliegenden Erfindung bevorzugten reaktiven Phosphorintermediate können am 3'-Ende, in der Mitte und am 5'-Ende an/in ein Oligonukleotid ein- oder mehrfach eingebaut werden. Durch Aktivierung kann ein mehrfach polyfunktioneller Thiolanker freigesetzt werden, der für eine mehrfache Anbindung z.B. eines Oligonukleotids an Oberflächen, an voraktivierte Oberflächen (z.B. Aldehyd, Maleinimid) oder an anderen polymeren Verbindungen (z.B. Proteine, PNA, RNA, LNA), die gegenüber Thiolen reaktive Gruppen enthalten, eingesetzt werden kann. Ebenso kann dieser polyfunktionelle Thiolanker zur gezielten mehrfachen Anbindung von Markierungen und Liganden an Molekülen oder Polymeren verwendet werden. Markierungen und Liganden sind z.B. enzymatische, chromogene, fluorogene, radioaktive, chemiluminiscente Label, in Nukleinsäureoligomere interkalierende Agentien, Metalle, Metallionen, Drugs, Hormone, Proteine, Peptide, nukleolytische und proteolytische Agentien, insbesondere bindende Agentien (wie z.B. Biotin, Antigene, Haptene, Antikörper, Rezeptoren) und andere Verbindungen von biologischem Interesse, die z.B. den Transport durch biologische Membranen beeinflussen oder die Löslichkeit von Oligonukleotiden verändern. Es gibt bekannte Verfahren, die es ermöglichen diese Liganden und Markierungen an Thiol-Gruppen anzukoppeln wie z.B. über Maleinimide, Aldehyde und Halogenacetylverbindungen (Means, G.M. und R.E. Feeney, Chemical Modification of Proteins, Holden-Day Inc., 1971; Feeney, R.E., Int. J. Peptide Protein Res., 29: 145-161, 1987; Eritja, R. et al., Tetrahedron, 47, 4113-4120, 1991).

Die Bindung zwischen der Oberfläche und dem Oligonukleotid mittels eines Thiolankers, vereinfacht als Oberfläche-S-Oligonukleotid ausgedrückt, ist bei mechanischer Beanspruchung, wie zum Beispiel bei Waschschritten, instabil. Einer der Vorteile der vorliegenden Erfindung ist die Möglichkeit, an einem Oligonukleotid einen Polyanker anzukoppeln, um damit die Anbindung des Oligonukleotids an Gold durch mehrere Au-S Bindungen zu optimieren. Dabei sind die Bedingungen zur Anbindung auf der Oberfläche wie z.B. die verwendete Salzkonzentration, das an der Oberfläche angelegte Potential oder die Art der vormodifizierten Oberfläche entscheidend. Weiterhin können Thiole, die bereits auf die Oberfläche aufgebracht wurden, mit diesem Polythiolanker verdrängt werden.

Weiterhin wird die Abspaltung der Schwefel-Schutzgruppe mit AgNO₃ durch die Anwendung von DMT als Schutzgruppe vermieden. Die DMT Schutzgruppe kann durch eine milde Säurebehandlung, die mit der Oligonukleotidchemie kompatibel ist, abgespalten werden. Die erfindungsgemäßen Verbindungen können mit den üblichen Standardbedingungen der Oligonukleotidchemie eingesetzt werden.

Die Phosphor-enthaltenden Verbindungen beinhalten Intermediate, die in der H-Phosphonat-, Phosphotriester-, Chlorophosphit- und Phosphoramidit-Methode zur Synthese von Oligonukleotiden eingesetzt werden können. Weiterhin können diese Intermediate, die Phosphodiester-Analoga wie Methylphosphonate, Methylphosphate, Phosphorthioate und Phosphoramidite einschließen (EP 0 523 978), für Modifikationen am 5'-, 3'-Ende und/oder in der Sequenz verwendet werden.

Gemäß einer bevorzugten Ausführungsform der vorliegenden Erfindung entspricht Y² der Formel **II** oder **III,** wobei X¹ ein Halogen und X² Methyl oder R⁷O- ist, wobei R⁷ ein Alkyl-, Cycloalkyl-, Aryl-Rest oder ein Cyanoderivat eines Alkyl-, Aryl-Rest ist, oder X² gleich R⁷O- und X¹ gleich -NR⁸R⁹ ist, wobei R⁸ und R⁹ unabhängig voneinander Alkyl-, Heteroalkyl, Cycloalkyl-, Aryl-Reste sind oder R⁸ und R⁹ miteinander verbunden sind, sodass sie mit dem N-Atom eine zyklische Struktur mit 4 bis 7 C-Atomen bilden, wobei ein C-Atom der zyklischen Struktur durch O oder S ersetzt sein kann, oder X³ = O⁻ und X⁴ = H oder R¹⁰O- ist, wobei R¹⁰ eine Schutzgruppe darstellt.

Die erfindungsgemäßen Verbindungen können auch an einem Trägermaterial (Solid Support) angebunden werden, wenn des Grundkörpers eine freie oder geschützte OH-Funktion aufweist. Es kann eine große Auswahl von Trägermaterialen benutzt werden wie z.B. Silika, Porasil C, Polystyrene, Controlled Pore Glass (CPG), Kieselgur, Poly(dimethylacrylamid), Poly(acrylmorpholid), Cellulose, Fractosil 500. Abhängig von der Art des Trägermaterials werden verschiedene Funktionalitäten als Anker verwendet. Substituierte Alkyl- oder Aryl-Silylverbindungen werden für Silicon-Trägermaterialien, wie Silika und Glas, verwendet, um einen Siloxan- oder Siloximin-Anker auszubilden. Ether, Ester, Amine, Amide, Sulfide, Sulfone und Phosphate können bei organischen Polymeren benutzt werden.

Für den Fall, dass Y² eine Gruppe der Formel (III) ist, wobei X³ gleich O⁻ und X⁴ gleich H sind, stellen die obigen Verbindungen H-Phosphonate dar und werden in der H-Phosphonat Methode für die Oligonukleotid Synthese verwendet (Sinha und Cook, Nucleic Acids Research (1988) 16:2659-2669). H-Phosphonate können zu Phosphitdiester, Phosphorthioaten, oder Phosphoramidaten umgesetzt werden, sobald sie am 5'-Ende des Oligonukleotids eingebaut sind (Miller et al., Nucleic Acids Res. (1983) 11:5189-5204, Eckstein, Ann. Rev. Biochem. (1985) 54:367-402).

Dementsprechend können die obigen Verbindungen, worin Y² eine Gruppe der Formel (III) ist mit X³ gleich O⁻ und X⁴ gleich R¹⁰O-, in der Phosphotriester Methode für die Oligonukleotid Synthese verwendet werden (Garegg, et al., Chemica Scripta (1985) 26:5).

Die Verbindungen, worin Y² eine Gruppe der Formel (II) darstellt, wobei X¹ gleich Chlor und X² gleich R⁷O- sind, ist ein Chlorophosphit und wird in der Chlorophosphit Technik für die Oligonukleotid Synthese verwendet (Wada et al., J. Org. Chem. (1991) 56:1243-1250).

Die Phosphoramidite mit Y² gleich der obigen Formel (II) werden im Rahmen der vorliegenden Erfindung besonders bevorzugt.

R¹ und R² sind gleiche oder verschiedene H oder Schwefel-Schutzgruppen. Bevorzugte Schwefel-Schutzgruppen sind beispielsweise Trityl, 4,4'-Dimethoxytrityl, 4-Monomethoxytrityl, 9-Fluorenylmethyl (Ponsati, B., et al., Tetrahedron, 46, 8255-8266, 1990), 9-Fluorenylmethoxycarbonyl, 2,4-Dinitrophenylethyl, 2,4,6-Trimethoxybenzyl (Munson, M.C. et al., J. Org. Chem., 57, 3013-3018, 1992), 4-Methoxybenzyl und Allyloxycarbonylaminomethyl (Kimbonguila, A.M., et al., Tetrahedron 55, 6931-6944, 1999). Besonders bevorzugt ist 4,4'-Dimethoxytrityl. Weitere Schutzgruppen kann man in Lloyd-Williams, P. et al., Chemical Approaches to the Synthesis of Peptides and Proteins, New York, CRC Press, 1997 finden. Die Schwefel-Schutzgruppen Trityl und Acetamidomethyl können mittels Iod Oxidation abgespalten werden (Kamber et al., Helvetica Chimica Acta, Vol. 63, No. 96, 899-915, 1980). Die Schwefel-Schutzgruppen 4,4'-Dimethoxytrityl und 4-Monomethoxytrityl können mittels AgNO₃ in MeOH abgespalten werden (Huang, Z. und Benner, S.A., Synlett, 83-84, 1993). Die 4,4'-Dimethoxytrityl Schwefel-Schutzgruppe kann auch unter milden Säurebedingungen (z.B. 2% Dichloressigsäure in Dichlormethan), die kompatibel mit der Oligonukleotidsynthese sind, abgespalten werden. Die große Auswahl an Schwefel-Schutzgruppen bietet die Möglichkeit orthogonale Schutzgruppen auszuwählen, deren Abspaltungsbedingungen kompatibel mit der Oligonukleotidsynthese sind, um verschiedene Labels einzuführen.

Gemäß einer bevorzugten Ausführungsform der vorliegenden Erfindung stellt der Rest R⁷ eine basenlabile Schutzgruppe dar.

Gemäß einer besonders bevorzugten Ausführungsform handelt es sich bei R⁷ um eine basenlabile Schutzgruppe ausgewählt aus ß-Cyanoethyl, ß-Nitroethyl, 2,2,2-Trichlorethyl, Methyl, 1,1-Dimethyl-2,2,2-thrichlorethyl, 2,2,2-Tribromethyl, Benzyl, o-Chlorphenyl, p-Nitrophenylethyl, 2-Methylsulfonylethyl und 1,1-Dimethyl-2-cyanoethyl.

Gemäß ganz besonders bevorzugten Ausführungsformen der vorliegenden Erfindung stellen R⁸ und R⁹ in dem Fall, dass R⁷ eine basenlabile Schutzgruppe ist, individuelle Alkyl-Reste bestehend aus 1-16 C-Atomen, Cycloalkyl-Reste bestehend aus 3-8 C-Atomen, Aryl-Reste bestehend aus 6-20 C-Atomen dar oder R⁸ und R⁹ sind miteinander verbunden, sodass sie mit dem N-Atom eine zyklische Struktur mit 4 bis 7 C-Atomen bilden, wobei ein C-Atom der zyklischen Struktur durch O oder S ersetzt sein kann. Dabei ist es besonders bevorzugt, wenn R⁸ und R⁹ unabhängig voneinander Alkylreste bestehend aus 1-6 C Atomen sind. Besonders bevorzugt ist es auch, wenn R⁸ und R⁹ Isopropyl, Butyl, Hexyl, Nonyl, Dodecyl, Hexadecyl, Cyclopropyl, Cyclobutyl, Cyclohexyl, Cyclooctyl, Phenyl, Tolyl, Benzyl, Xylyl, Naphthyl, Morpholino, Piperidinyl oder Thiomorpholino sind.

Weitere Schutzgruppen R⁸ und R⁹ sind in Green, T.W., Protective Groups in Organic Chemistry, New York: Wiley & Sons, 1981 ausgeführt.

Die vorliegende Erfindung umfasst auch Verbindungen der Formel 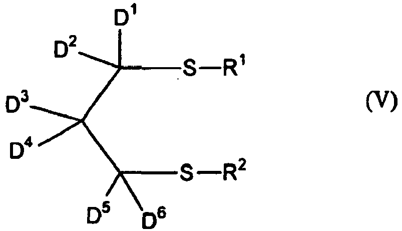 wobei D¹, D², D³ und D⁵ gleich H sind, D⁶ gleich Y² ist, D⁴ ein Alkylrest mit 1-22 C-Atomen, ein Heteroalkylrest mit 1-22 C-Atomen, ein Cycloalkylrest mit 1-22 C-Atomen ist und Schutzgruppe-Y¹ umfasst, oder D⁴ gleich Schutzgruppe-Y¹ ist, R¹ und R² gleiche oder verschiedene H oder Schwefel-Schutzgruppen sind, wobei die beiden S-Atome auch eine Disulfid-Brücke ausbilden können und in diesem Fall R¹, R² nicht vorhanden sind, Schutzgruppe-Y¹ gleich Schutzgruppe-S, Schutzgruppe-NH, Schutzgruppe-NR⁴, Schutzgruppe-O, Schutzgruppe-S-S, Schutzgruppe-OOC ist, Schutzgruppe gleich 9-Fluorenylmethoxycarbonyl oder Dimethoxytrityl ist, Y² gleich -OH, -NH₂, -NHR³, -NR³R⁴, -COOH, -COCI, -COOCO-R⁶, -CONH₂,-CONHR³, -COOR³, -SO₃H, -SO₃Cl, -SH, -S-SR³, -CHO, -COR³, -C₂H₃O, Halogen,-N₃, -NH-NH₂, -NCO, -NCS ist, wobei R³ Alkyl, Heteroalkyl, Aryl, Cycloalkyl oder eine Schutzgruppe ist, R⁴ eine Schutzgruppe ist, wobei R⁴ und R³ gleich oder verschieden sein können, R⁵ Alkyl, Aryl, Cycloalkyl ist, und R⁶ Alkyl, Heteroalkyl, Aryl oder Cycloalkyl ist, wobei Y² auch eine Gruppe der Formel (II) oder (III) sein kann, 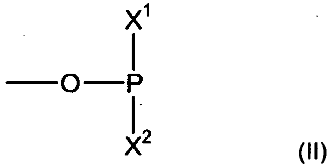 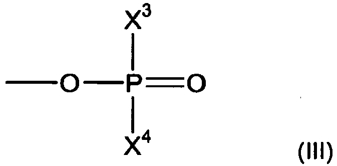 wobei X¹ ein Halogen oder ein substituiertes Amin ist, X² ein Alkyl-, Alkoxy-, Aryloxy-Rest oder ein Cyanoderivat eines Alkyl-, Alkoxy-, Aryloxy-Restes ist, X³ ein Halogen, eine Aminofunktion oder Sauerstoff ist und X⁴ ein Alkyl-, Alkoxy-, Aryloxy-Rest ist oder X⁴ gleich H ist für den Fall, dass X³ = Sauerstoff ist.

Die vorliegende Erfindung umfasst auch Verbindungen der Formel 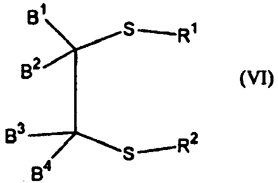 wobei B¹, B² und B³ gleich H sind, B⁴ ein Alkylrest mit 1-22 C-Atomen, ein Heteroalkylrest mit 1-22 C-Atomen, ein Cycloalkylrest mit 1-22 C-Atomen ist und die Gruppen Schutzgruppe-Y¹ und Y² umfasst, R¹ und R² gleiche oder verschiedene H oder Schwefel-Schutzgruppen sind, wobei die beiden S-Atome auch eine Disulfid-Brücke ausbilden können und in diesem Fall R¹, R² nicht vorhanden sind, Schutzgruppe-Y¹ gleich Schutzgruppe-S, Schutzgruppe-NH, Schutzgruppe-NR⁴, Schutzgruppe-O, Schutzgruppe-S-S, Schutzgruppe-OOC ist, Schutzgruppe gleich 9-Fluorenylmethoxycarbonyl oder Dimethoxytrityl ist, Y² gleich -OH, -NH₂, -NHR³,-NR³R⁴, -COOH, -COCl, -COOCO-R⁶, -CONH₂, -CONHR³, -COOR³, -SO₃H, -SO₃Cl, -SH, -S-SR³, -CHO, -COR³, -C₂H₃O, Halogen, -N₃, -NH-NH₂, -NCO, -NCS ist, wobei R³ Alkyl, Heteroalkyl, Aryl, Cycloalkyl oder eine Schutzgruppe ist, R⁴ eine Schutzgruppe ist, wobei R⁴ und R³ gleich oder verschieden sein können, R⁵ Alkyl, Aryl, Cycloalkyl ist, und R⁶ Alkyl, Heteroalkyl, Aryl oder Cycloalkyl ist, wobei Y² auch eine Gruppe der Formel (II) oder (III) sein kann, 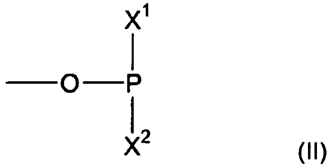 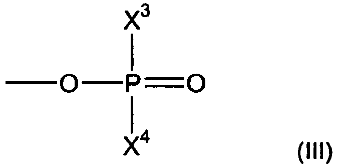 wobei X¹ ein Halogen oder ein substituiertes Amin ist, X² ein Alkyl-, Alkoxy-, Aryloxy-Rest oder ein Cyanoderivat eines Alkyl-, Alkoxy-, Aryloxy-Restes ist, X³ ein Halogen, eine Aminofunktion oder Sauerstoff ist und X⁴ ein Alkyl-, Alkoxy-, Aryloxy-Rest ist oder X⁴ gleich H ist für den Fall, dass X³ = Sauerstoff ist.

Die vorliegende Erfindung umfasst auch die Verwendung der erfindungsgemäßen Verbindungen zur Modifikation von Oligomeren. Daneben umfasst die vorliegende Erfindung auch die Verwendung der erfindungsgemäßen Verbindungen zur Immobilisierung von modifizierten Oligomeren auf Oberflächen. Außerdem umfasst die vorliegende Erfindung auch die Verwendung der erfindungsgemäßen Verbindungen zur Anbindung von enzymatischen, chromogenen, fluorogenen, radioaktiven oder chemiluminiscenten Labeln, von in Nukleinsäureoligomere interkalierenden Substanzen, von Metallen, von Metallionen, Hormonen, Proteinen, Peptiden, nukleolytischen und proteolytischen Agentien, Biotin, Antigenen, Haptenen, Antikörpern oder Rezeptoren an Moleküle oder Oligomere. Schließlich umfasst die vorliegende Erfindung auch die Verwendung der erfindungsgemäßen Verbindungen bei der automatischen Synthese von Oligomeren.

Im Rahmen der genannten Verwendungen der erfindungsgemäßen Verbindungen wird es besonders bevorzugt, dass es sich bei den Oligomeren um Oligonukleotide, Polypeptide, PNA oder LNA (Locked Nucleic Acid) handelt.

Die Synthese von Oligonukleotiden, die mit den erfindungsgemäßen Verbindungen modifiziert werden, erfolgt in Lösung oder vorzugsweise an fester Phase, gegebenenfalls unter Zuhilfenahme eines automatischen Synthesegeräts.

### Kurze Beschreibung der Zeichnungen

Es zeigen

| | |
|---|---|
| Figur 1 | ein Syntheseschema gemäß Beispiel 4; |
| Figur 2 | eine SPR-Kinetik zur Immobilisierung von Oligonukleotiden gemäß Beispiel 13 auf einer Goldoberfläche; |
| Figur 3 | einen Stabilitätstests eines Oligonukleotids gemäß Beispiel 13 auf einer Goldoberfläche; |
| Figur 4 | Figur 4.1 und Figur 4.2 zeigen die Ergebnisse von Square-Wavevoltammetrischen Messungen zur Überprüfung der Hybridisierbarkeit der Oligonukleotide gemäß Beispiel 13 auf einer Goldoberfläche; |
| | Figur 4.3 und Figur 4.4 zeigen die Ergebnisse cyclovoltammetrischer Messungen zur Quantifizierung der Hybridisierbarkeit der Oligonukleotide gemäß Beispiel 13 auf einer Goldoberfläche; |
| Figur 5 | zeigt das Ausmaß der Oberflächenbelegung durch die Oligonukleotide gemäß Beispiel 13 auf einer Goldoberfläche durch den Integrationen der Peakflächen von Cyclovoltammogrammen; |
| Figur 6 | ein Syntheseschema gemäß Beispiel 15; |
| Figur 7 | ein Syntheseschema gemäß Beispiel 16; |
| Figur 8 | ein Syntheseschema gemäß Beispiel 17; |
| Figur 9 | ein Syntheseschema gemäß Beispiel 18. |

### Wege zur Ausführung der Erfindung

### Beispiel 1: Herstellung von 3-O-(4,4'-Dimethoxytrityl)-1,4-bis-(4,4'-dimethoxitrityl)-sulfanyl-butan-2-ol

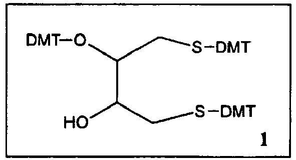

Unter Argonatmosphäre werden in einem 250 ml Rundkolben 2.0 g (12.9 mmol) 1,4-Dithio-butan-2,3-diol unter Rühren in 35 ml wasserfreiem Pyridin gelöst. Zu der klaren Lösung werden 15.3 g (45.15 mmol) DMT-Cl (4,4'-Dimethoxytrityl Chlorid) gegeben. Nach 2 Stunden unter Rühren bei Raumtemperatur wird das Reaktionsgemisch auf 50°C erhitzt und über Nacht gerührt. Danach wird MeOH (2 ml) zugesetzt und 10 min gerührt. Anschließend wird im Hochvakuum eingeengt, der Rückstand in 200 ml DCM aufgenommen und je einmal mit 1 mol/l NaHCO₃-Lösung und NaCl-Lösung ausgeschüttelt. Die organische Phase wird über Na₂SO₄ wasserfrei getrocknet, abfiltriert und abgezogen. Zur Aufreinigung des Rohproduktes wird an Kieselgel 60 chromatographiert (Laufmittel: Ethylacetat/n-Heptan/1%Et₃N). Die produktenthaltenden Fraktionen werden gesammelt und das Lösungsmittel restlos im Vakuum abgezogen. DC (Kieselgel, EtOAc/n-Heptan = 1:2, +1 % Et₃N): R_{f} = 0.20.

Man erhält 5.90 g (43.2% der Theorie) eines gelblichen, schaumigen Rückstandes.

¹³C-NMR (CD₃Cl) δ (ppm): 33.21 (C-4), 35.82 (C-1), 55.17 (OCH₃), 65.50 und 65.82 (C-2 und C-3), 71.52 und 75.02 (S-Cq DMT), 87.03 (O-Cq DMT), 113.01, 126.32, 127.71, 129.30, 131.03, 136.21, 137.13, 145.25, 145.76, 146.10, 157.72, 158.61 (Cₐᵣₒₘ DMT).

¹H-NMR (CD₃Cl) δ (ppm): 2.05-2.21 (m, 4H, CH₂-1 und CH₂-4), 3.17 (m, 1H, H-3), 3.65 (m, 1H, H-2), 6.72-7.38 (m, 39Hₐᵣₒₘ DMT).

### Beispiel 2: Herstellung von 3-O-(4,4'-Dimethoxytrityl)-1,4-bis-(4,4'-dimethoxitrityl)-sulfanyl-butan-2-O-(2-cyanoethyl)-N,N'-diisopropylphosphoramidit

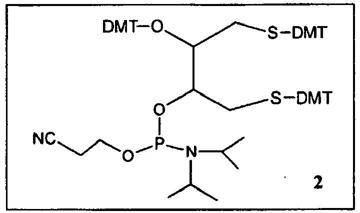

Unter Argonatmosphäre werden 503 mg (0.47 mmol) 3-O-(4,4'-Dimethoxytrityl)-1,4-bis-(4,4'-dimethoxitrityl)sulfanyl-butan-2-ol in 4 ml wasserfreiem ACN gelöst. Die Lösung wird mit Eis gekühlt und unter Rühren werden 753.2 µl (4.4 mmol) N,N'-Diisopropylethylamin tropfenweise gegeben. Dazu werden mit einer Spritze 295 µl (1.32 mmol) Chlor-(2-cyanoethoxy)(diisopropylamino)-phosphin getropft. Nach 1,5 Stunden unter Rühren bei Raumtemperatur wird das Reaktionsgemisch mit 30 ml DCM verdünnt und je einmal mit einer 1 mol/l NaHCO₃-Lösung und einer gesättigten Natriumchloridlösung ausgeschüttelt. Die organische Phase wird über Na₂SO₄ wasserfrei getrocknet, abfiltriert und eingeengt. Zur Reinigung des Rohprodukts wird über Kieselgel chromatographiert (Laufmittel: Gradient Ethlyacetat/n-Heptan 3:1 bis 2:1 in Gegenwart von 1% Et₃N). Die beiden Diastereomere können sowohl im DC als auch im ³¹P-NMR unterschieden werden: DC (Kieselgel, EtOAc/n-Heptan = 1:2, +1 % Et₃N): R_{f} (2 Diastereomere) = 0.20; 0.27

Man erhält 331.3 mg (55.4% der Theorie) eines weißen, schaumigen Rückstands.

³¹P-NMR (CD₃Cl) δ (ppm): 149.65, 148.59

MS (EI): 303 (DMT⁺), 1003.1 (M+Na⁺)

### Beispiel 3: Herstellung von 5-O-(4,4'-Dimethoxytrityl)-(1,2)-dithian-4-ol

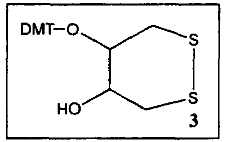

Zu einer Lösung von 2 g (1.88 mmol) 3-O-(4,4'-Dimethoxytrityl)-1,4-bis-(4,4'-dimethoxitrityl)sulfanyl-butan-2-ol und 5 ml Pyridin in 100 ml DCM wird bei Raumtemperatur eine Lösung von l₂ in DCM (9.4 mmol l₂ in 120 ml DCM) gegeben. Nach 10 min. Rühren werden 200 ml einer 0.5 N Na₂S₂O₃ Lösung zugegeben. Die Phasen werden getrennt, die organische Phase dreimal mit H₂O extrahiert und die vereinigten organischen Phasen über Na₂SO₄ getrocknet. Das Lösungsmittel wird abgedampft und der verbleibende Schaum an Kieselgel chromatographiert (Laufmittel: Gradient von 10-30% EtOAc in n-Heptan in Gegenwart von 1 % Et₃N). DC (Kieselgel, EtOAc/n-Heptan = 1:2, +1% Et₃N): R_{f} (2 Diasteromere) = 0.31; 0.40.

Man erhält 710.7 mg entsprechend 83.6% der Theorie eines gelblichen Öls.

¹³C-NMR (CD₃Cl) δ (ppm): 32.76 (C-6), 41.83 (C-3), 55.24 (OCH₃), 67.21 (C-5), 72.32 (C-4), 87.25 (Cq DMT), 113.16, 126.64, 127.89, 129.39, 130.61, 136.94, 145.14, 158.09 (Carom DMT).

¹H-NMR (CD₃Cl) δ (ppm): 2.75 (m, 2H, CH₂-6), 3.03 (m, 2H, CH₂-3), 3.69 (m. 2H, H-4, H-5), 3.79 (s, 3H, OCH₃), 6.83-7.52 (m, 13Hₐᵣₒₘ DMT).

MS (Electrospray ionisation in MeOH): 303 (DMT⁺), 477 (M+Na⁺).

### Beispiel 4: Herstellung von 5-O-(4,4'-Dimethoxytrityl)-(1,2)-dithian-4-O-(2-cyanoethyl)-N,N'-diisopropylphosphoramidit

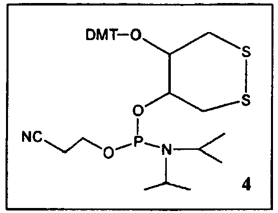

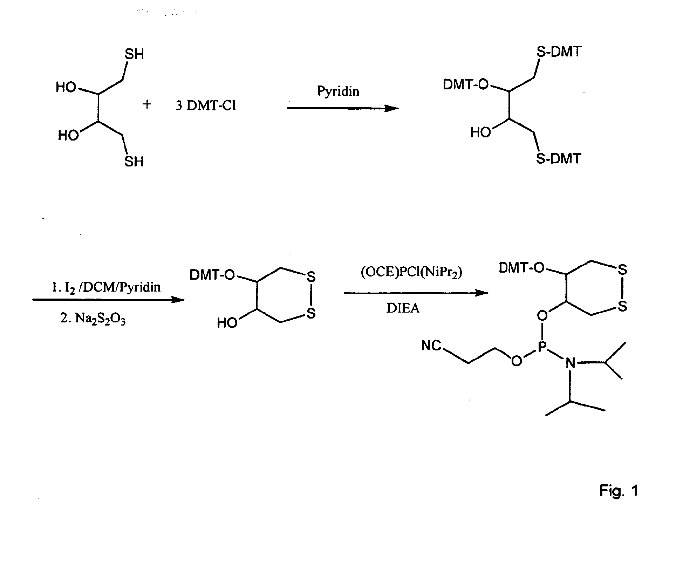

Eine schematische Synthese-Übersicht ist in der Figur 1 dargestellt.

Unter Argonatmosphäre werden 500 mg (1.1 mol) 5-O-(4,4'-Dimethoxytrityl)-(1,2)-dithian-4-ol in 8 ml wasserfreiem DCM gelöst. Die Lösung wird mit Eis gekühlt und unter Rühren werden 753.2 µl (4.4 mmol) N,N'-Diisopropylethylamin tropfenweise zugegeben. Dazu werden mit einer Spritze 295 µl (1.32 mmol) Chlor-(2-cyanoethoxy)(diisopropylamino)-phosphin getropft. Nach 1 Stunde unter Rühren bei Raumtemperatur wird das Reaktionsgemisch mit 50 ml DCM verdünnt und je einmal mit 1 mol/l NaHCO₃-Lösung und gesättigter Natriumchloridlösung ausgeschüttelt. Die organische Phase wird über Na₂SO₄ wasserfrei getrocknet, abfiltriert und eingeengt. Zur Reinigung des Rohprodukts wird über Kieselgel chromatographiert (Laufmittel: Gradient von 5-15% EtOAc in n-Heptan in Gegenwart von 1 % Et₃N). Die beide Diastereomere können sowohl im DC als auch im ³¹P-NMR unterschieden werden: DC (Kieselgel, EtOAc/n-Heptan = 1:2, +1% Et₃N): R_{f} (2 Diastereomere) = 0.31; 0.40.

Man erhält 459.6 mg (63.8% der Theorie) des gewünschten Produkts als gelbliches Öl.

³¹P-NMR (CD₃Cl) δ (ppm): 148.33, 150.19.

MS (EI): 303 (DMT⁺), 655 (M), 677 (M+Na⁺)

### Beispiel 5: Herstellung von 1,4-bis-(4,4'-Dimethoxitrityl)sulfanyl-butan-2,3-diol

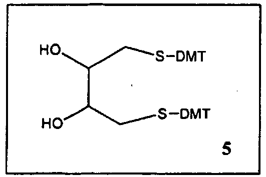

Unter Argonatmosphäre werden in einem 250 ml Rundkolben 2.2 g (14.3 mmol) 1,4-Dithio-butan-2,3-diol unter Rühren in 40 ml wasserfreiem Pyridin gelöst. Zu der klaren Lösung werden 9.93 g (29.3 mmol) DMT-Cl (4,4'-Dimethoxytrityl Chlorid) gegeben. Nach 2 Stunden unter Rühren bei Raumtemperatur werden 2 ml MeOH zugegeben und 5 min nachgerührt. Das Lösungsmittel wird abgedampft und der Rückstand in 50 ml DCM gelöst und je einmal mit einer NaHCO₃-Lösung und einer NaCl-Lösung extrahiert. Die organische Phase wird über Na₂SO₄ getrocknet, abfiltriert und eingeengt. Der verbleibende Schaum wird an Kieselgel chromatographiert (Laufmittel: Gradient von 10-30% Ethylacetat/n-Heptan in Gegenwart von 1%Et₃N). Die produktenthaltenden Fraktionen werden gesammelt und das Lösungsmittel restlos im Vakuum abgezogen. DC (Kieselgel, EtOAc/n-Heptan = 1:2, +1 % Et₃N): R_{f} = 0.35.

Man erhält 8.66 g (79.8% der Theorie) eines weißen, schaumigen Rückstands.

¹³C-NMR (CD₃Cl) δ (ppm): 34.83 (C-1, C-4), 55.20 (OCH₃ DMT), 65.94 (C-2, C-3), 71.70 (Cq DMT), 113.23, 126.61, 127.95, 129.40, 130.62, 136.96, 145.14, 158.09 (Cₐᵣₒₘ DMT). ¹H-NMR (CD₃Cl) δ (ppm): 2.01 (m, 2H, OH-2, OH-3), 2.35 (m, 4H, C-1, C-4), 3.05 (m, 2H, C-2, C-3), 3.73 (s, 12H, OCH₃), 6.78-7.45 (m, 26Hₐᵣₒₘ DMT).

### Beispiel 6: Herstellung von 3-O-(9-Fluorenylmethoxycarbonyl)-1,4-bis-(4,4'-dimethoxitrityl)sulfanyl-butan-2-ol

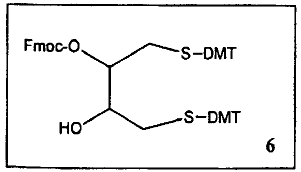

Unter Argonatmosphäre werden zu einer Lösung von 1 g (1.32 mmol) 1,4-bis-(4,4'-Dimethoxitrityl)sulfanyl-butan-2,3-diol in 10 ml wasserfreiem Pyridin bei Raumtemperatur 407 mg (1.58 mmol) 9-Fluorenylmethylchloroformat gegeben. Das Reaktionsgemisch wird über Nacht bei Raumtemperatur gerührt. Danach werden 500 µl MeOH zugegeben und für 10 min gerührt. Nach Einengen des Lösungsmittels wird der Rückstand in 30 ml DCM aufgenommen und einmal mit einer NaCl-Lösung extrahiert. Nach Trocknen der organischen Phase über Na₂SO₄ und Abdampfen des Lösungsmittels wird das Rohprodukt an Kieselgel chromatographiert (Laufmittel: Ethylacetat/n-Heptan = 3:1). DC (Kieselgel, EtOAc/n-Heptan = 1:2): R_{f} = 0.25.

Man erhält 303 mg (23.4% der Theorie) eines weißen, schaumigen Rückstands.

¹³C-NMR (CD₃Cl) δ (ppm): 31.68 (C-4), 35.06 (C-1), 55.20 (OCH₃ DMT), 50.36 (CH-Fmoc), 65.31 und 65.45 (C-2 und C-3), 66.77 (CH₂-Fmoc), 70.38 (2S-C-DMT), 113.23, 120.05, 124.70, 127.06, 127.6, 128.08, 129.22, 136.24, 136.82, 141.51, 143.31, 144.35, 145.01, 154.36, 158.10, 158.3 (Cₐᵣₒₘ DMT und Fmoc).

¹H-NMR (CD₃Cl) δ (ppm): 2.10-2.45 (m, 4H, CH₂-1, CH₂-4), 3.73 (d, 12H, OCH₃ DMT), 3.98-4.35 (m, 2H, H-3, H-2), 6.78-7.82 (m, 34H, Hₐᵣₒₘ DMT und Fmoc).

MS (Electrospray ionisation): 303.2 (DMT⁺), 1283.3 (M+Na⁺)

### Beispiel 7: Herstellung von 5-O-(9-Fluorenylmethoxycarbonyl)-(1,2)-dithian-4-ol

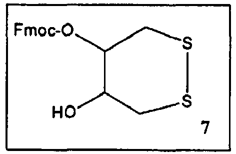

Zu einer Lösung von 1.23 g (1.25 mmol) 3-O-(9-Fluorenylmethyloxycarbonyl)-1,4-S,S'-bis-(4,4'-dimethoxitrityl)-butan-2-ol in 50 ml DCM wird bei Raumtemperatur eine Lösung von l₂ in DCM (6.25 mmol l₂ in 60 ml DCM) gegeben. Nach 10 min werden unter starkem Rühren 100 ml einer 0.5 N Na₂S₂O₃-Lösung zugegeben. Die Phasen werden getrennt, die organische Phase dreimal mit H₂O extrahiert und die vereinigten organischen Phasen über Na₂SO₄ getrocknet. Das Lösungsmittel wird abgedampft und der verbleibende Schaum an Kieselgel chromatographiert (Laufmittel: Gradient von 10-30% EtOAc in n-Heptan). DC (Kieselgel, EtOAc/n-Heptan = 1:2): R_{f} = 0.20.

Man erhält 212 mg (45.3% der Theorie) eines gelblichen Öls.

¹³C-NMR (CD₃Cl) δ (ppm): 34.51 (C-6), 41.86 (C-3), 46.74 (CH-Fmoc), 65.18 (CH₂-Fmoc), 70.11 (C-5), 72.21 (C-4), 120.06, 125.07, 127.59, 127.98, 141.33, 143.18 (Cₐᵣₒₘ Fmoc)

¹H-NMR (CD₃Cl) δ (ppm): 2.98 (m, 2H, CH₂-6), 3.09 (m, 2H, CH₂-3), 3.72 (m, 2H, H-4, H-5), 7.28-7.80 (m, 8Hₐᵣₒₘ Fmoc).

### Beispiel 8: Herstellung von 3-O-(9-Fluorenylmethoxycarbonyl)-1,4-bis-(4,4'-dimethoxitrityl)sulfanyl-butan-2-O-(2-cyanoethyl)-N,N'-diisopropylphosphoramidit

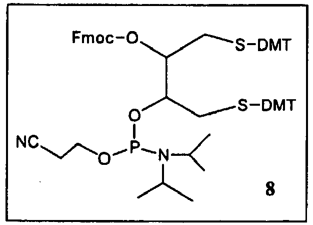

Unter Argonatmosphäre werden 460 mg (0.47 mmol) 3-O-(9-Fluorenylmethoxycarbonyl)-1,4-bis-(4,4'-dimethoxitrityl)sulfanyl-butan-2-ol in 5 ml wasserfreiem DCM gelöst. Zur eisgekühlten Lösung werden unter Rühren 322 µl (1.88 mmol) N,N'-Diisopropylethylamin tropfenweise gegeben. Dazu werden mit einer Spritze 136.5 µl (0.61 mmol) Chlor-(2-cyanoethoxy)(diisopropylamino)-phosphin getropft. Nach einer Stunde unter Rühren bei Raumtemperatur wird das Reaktionsgemisch mit 50 ml DCM verdünnt und je einmal mit 1 mol/l NaHCO₃-Lösung und gesättigter Natriumchloridlösung ausgeschüttelt. Die organische Phase wird über Na₂SO₄ wasserfrei getrocknet, abfiltriert und eingeengt. Zur Reinigung des Rohprodukts wird über Kieselgel chromatographiert (Laufmittel: Gradient von 5-20% EtOAc in n-Heptan in Gegenwart von 1% Et₃N). Die beiden Diastereomere können sowohl im DC als auch im ³¹P-NMR unterschieden werden: DC (Kieselgel, EtOAc/n-Heptan = 1:2, +1 % Et₃N): R_{f} (2 Diastereomere) = 0.26; 0.29

Man erhält 270 mg entsprechend 48.7% der Theorie eines weißen, schaumigen Rückstands.

³¹P-NMR (CD₃Cl) δ (ppm): 149.35, 150.29.

### Beispiel 9: Herstellung von 5-O-(9-Fluorenylmethoxycarbonyl)-(1,2)-dithianyl-4-O-(2-cyanoethyl)-N,N'-diisopropylphosphoramidit

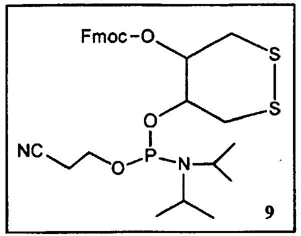

Unter Argonatmosphäre werden 500 mg (1.33 mmol) 5-O-(9-Fluorenylmethoxycarbonyl)-(1,2)-dithianyl-4-ol in 5 ml wasserfreiem DCM gelöst. Zur eisgekühlten Lösung werden unter Rühren 914 µl (5.32 mmol) N,N'-Diisopropylethylamin tropfenweise gegeben. Dazu werden mit einer Spritze 387 µl (1.73 mmol) Chlor-(2-cyanoethoxy)(diisopropylamino)-phosphin getropft. Nach 1.5 Stunden unter Rühren bei Raumtemperatur wird das Reaktionsgemisch mit 50 ml DCM verdünnt und je einmal mit 1 mol/l NaHCO₃-Lösung und gesättigter Natriumchloridlösung ausgeschüttelt. Die organische Phase wird über Na₂SO₄ wasserfrei getrocknet, abfiltriert und eingeengt. Zur Reinigung des Rohprodukts wird über Kieselgel chromatographiert (Laufmittel: Gradient von 5-20% EtOAc in n-Heptan in Gegenwart von 1 % Et₃N). Die beiden Diastereomere können sowohl im DC als auch im ³¹P-NMR unterschieden werden: DC (Kieselgel, EtOAc/n-Heptan = 1:2, +1% Et₃N): R_{f} (2 Diastereomere) = 0.22; 0.3

Man erhält 295 mg entsprechend 38.7% der Theorie eines weißen, schaumigen Rückstands.

³¹P-NMR (CD₃Cl) δ (ppm): 149.04, 150.32

### Beispiel 10: Festphasensynthese thiol-modifizierter Oligonukleotide mit Hilfe der Verbindung gemäß Beispiel 4 nach der Phosphoramidit-Methode

Die Oligodesoxyribonukleotidsynthesen wurden im 1 µmol Maßstab mittels der Festphasen-Phosphoramidit-Technik an einem automatisierten DNA/RNA-Synthesizer Modell 384 B (Applied Biosystems) mit Hilfe von ^{®}CPG (Controlled Pore Glass), das die erste Nukleosid-Einheit über das 3'-Ende gebunden enthält, durchgeführt. Hierzu wird das Synthese-Gerät z.B. mit einer Reaktionssäule bestückt, die mit einem mit Nukleobasen vorbelegten Trägermaterial beschickt wurde und in einem ersten Reaktionsschritt die 5'-OH-Schutzgruppe (4,4'-Dimethoxytrityl) durch Behandlung mit einer 2%igen Dichloressigsäure-Lösung in Dichlormethan abgespalten. Nach Waschen der Säule mit Acetonitril erfolgt die Kupplung des nächsten Bausteins, der auch das modifzierte Amidit aus Beispiel 4 sein kann, durch Aktivierung mit Tetrazol in ACN an die freie 5'-OH-Funktion. Zum Einbau des modifizierten Amidits wurde ein jeweils 10 minütiger Doppelkupplungsschritt verwendet. Das noch trivalent vorliegende P-Atom wird nach erneutem Waschen durch Oxidation mit einer Lösung von Iod in THF/Lutidin/H₂O in das natürliche pentavalente Phosphat überführt. Der nachfolgende Capping-Schritt mit Essigsäureanhydrid/1-Methylimidazol blockiert durch Acetylierung freie 5'-OH Gruppen. Dadurch wird die Bildung von Fehlsequenzen unterdrückt. Nach dem Waschen beginnt mit erneuter Abspaltung der 5'-O-Dimethoxytrityl-Schutzgruppe der Synthesezyklus von vorne. In dieser Weise wird das modifizierte Oligonukleotid aufgebaut. Die letzte DMT-Gruppe wurde nicht abgespalten. Nach beendeter Synthese wurde durch Behandlung mit konzentrierter wässriger Ammoniaklösung das am Träger gebundene Oligonukleotid freigesetzt. Die Schutzgruppen an den Heterozyklen wurden im gleichen Medium innerhalb von 16 h bei 37°C entfernt. Die Proben wurden im Vakuum auf etwa 200 µl eingeengt und mittels HPLC aufgereinigt.

### Beispiel 11: Festphasensynthese thiol-modifizierter Oligonukleotide mit Hilfe der Verbindung gemäß Beispiel 9 nach der Phosphoramidit-Methode

Die Oligomer-Synthese erfolgt wie in Beispiel 10 beschrieben. Zur Abspaltung der Fmoc Gruppe wird das Oligonukleotid am Träger mit einer 0.5 mol/l DBU Lösung in Acetonitril behandelt (4 x 1 ml 0.5 mol/l DBU/ACN in 2 min). Die Aufarbeitung erfolgt analog Beispiel 10.

### Beispiel 12: Festphasensynthese thiol-modifizierter Oligonukleotide mit Hilfe der Verbindung gemäß Beispiel 2 nach der Phosphoramidit-Methode

Der Oxidationsschritt bei der Oligomer-Synthese wurde mit einer 0.1 mol/l lod-Lösung und mit verlängerten Reaktionszeiten (1 min) durchgeführt. Der weitere Synthese-Zyklus und die Aufarbeitung der Oligonukleotide erfolgt analog Beispiel 10.

### Beispiel 13: HPLC-Aufreinigung der tritylgeschützten Oligonukleotide

Im ersten Reinigungsschritt wurden die DMT-geschützten Oligomere über HPLC an einer RP-C18-Kieselgel-Säule aufgereinigt (Laufmittel: 0.1 mol/l Triethylammoniumacetat-Puffer, Acetonitril). Die Oligomere wurden mit 100 µl einer 80% Essigsäure Lösung versetzt und bei Raumtemperatur 20 min geschüttelt. Zu dieser Lösung werden 100 µl H₂O und 60 µl 3 mol/l NaAc-Lösung gegeben. Die Oligonukleotide wurden mit 1.5 ml EtOH versetzt und vollständig bei -20 °C (20 min) gefällt. Nach Abzentrifugieren und Abdekantieren des Ethanols wird das Pellet in Vakuum getrocknet. Die Charakterisierung der Oligomere erfolgt mittels MALDI-TOF MS. Tabelle 1 zeigt die HPLC-Retentionszeiten der synthetisierten Oligonukleotide.

**Tabelle 1**

| Oligomer (5'->3'-Richtung) (Sequenz) | Retentionszeiten (min) mit DMT |
|---|---|
| Oligonukleotid 1: | |
| 5'-XAGG TGA CTG TGT TAT CCG CA-3' | 10.05 |
| Oligonukleotid 2: | |
| 5'-XXAGG TGA CTG TGT TAT CCG CA-3' | 11.30 |
| Oligonukleotid 3: | |
| 5'-XXXAGG TGA CTG TGT TAT CCG CA-3' | 11.72 |
| wobei X der Verbindung 4 entspricht | |
| | |
| 5'-XT10-3' | 21.12 |
| 5'-XXT10-3' | 21.52 |
| wobei X der Verbindung 2 entspricht | |

### Beispiel 14: Immobilisierungsexperimente mit den Oligonukleotiden 1, 2 und 3 gemäß Beispiel 13

### Präparation der Einzelstrang-DNA-Monoschicht:

### a) Reinigung der Au-Elektroden:

Zur Reinigung der Goldelektroden wurden die goldbedampften Glas-Objektträger 30 Sekunden in eine Mischung (3:1) aus konzentrierter Schwefelsäure und 30%iger wässriger Wasserstoffperoxidlösung getaucht, gründlich mit Reinstwasser abgespült und anschließend 15 Minuten in Ethanol aufbewahrt.

### b) Immobilisierung der Oligonukleotide 1, 2 und 3 auf der Au-Oberfläche :

Die Immobilisierung der Oligonukleotide auf der Au-Oberfläche erfolgte durch Selbstassemblierung aus einer 30 µmol/l Lösung der Oligonukleotide in Kaliumphosphatpuffer (500 mmol/l, pH 7) über Nacht, gefolgt von gründlichem Spülen mit Kaliumphosphatpuffer.

Zur Überprüfung der Immobilisierungseffektivität wurde die Oberflächen-plasmonen-Resonanz-Spektroskopie (SPR) eingesetzt, welche sehr sensitiv auf die mit Adsorptions- und Desorptionsvorgängen verbundenen Änderungen des Brechungsindex in Oberflächennähe reagiert. Die Änderung des Resonanzwinkels ΔΘ ist dabei proportional zum Massenzuwachs bzw. -verlust auf der Oberfläche. Die Experimente wurden auf einem Biosuplar **II** von Analytical µ-Systems (Regensburg) durchgeführt.

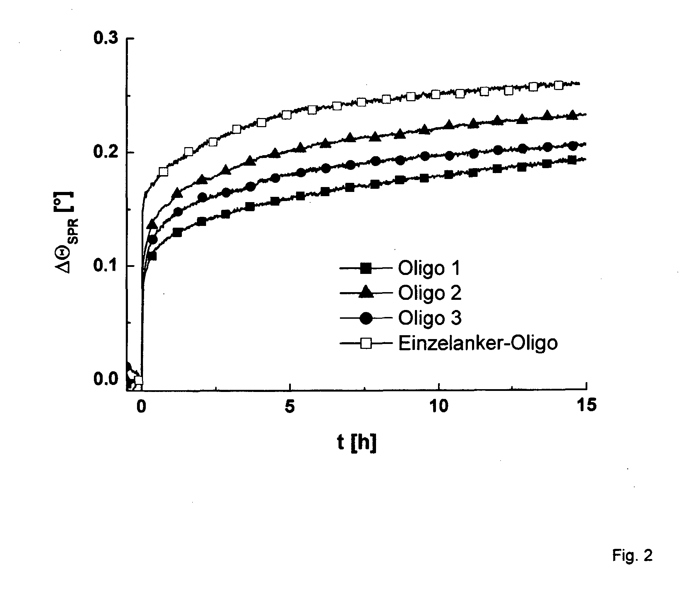

Die Figur 2 zeigt die SPR-Kinetiken zur Immobilisierung von Oligo 1 (■), Oligo 2 (▲) und Oligo 3 (●) auf einer Au-Oberfläche (c = 30 µM in 500 mM Kaliumphosphatpuffer, pH 7). Aus der Auftragung ist zu ersehen, dass die Oligonukleotide 1, 2 und 3 alle mit annähernd identischer Kinetik an die Oberfläche anbinden. Die adsorbierte Stoffmenge liegt für alle Oligonukleotide unter der von vergleichbaren Einzelanker-Oligonukleotiden (□) (H₂N-C₆-TCG TCA CTG TCA GTG TCA GA-[C₃-S-S-C₃-OH] mit C₃ = (CH₂)₃ und C₆ = (CH₂)₆) und spiegelt somit den erhöhten Grundflächenbedarf pro DNA-Strang wider.

### c) Nachbelegung mit Alkanthiolen:

Die Oligonukleotid-modifizierte Au-Oberfläche wurde zur Aufrichtung der DNA und zur Passivierung der Zwischenräume mit kurzkettigen Alkanthiolen nachbelegt. Die 30-minütige Nachbelegung erfolgte aus einer 1 mM Lösung des Alkanthiols (Propanthiol oder Hydroxypropanthiol) in obigem Kaliumphosphatpuffer mit 1% Ethanol, gefolgt von gründlichem Spülen mit Kaliumphosphatpuffer.

### Stabilitätstests:

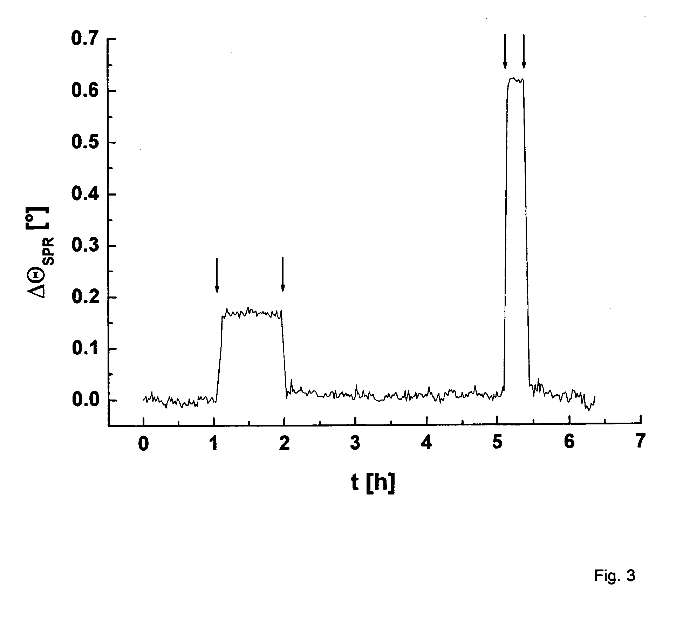

Die Überprüfung der Stabilität der Anbindung erfolgte durch Inkubation der Oberfläche mit Phosphatpuffer (pH 9) und durch Einstellen der Dehybridisierungsbedinungen (2 mol/l NaOH) unter SPR-Kontrolle. Die Figur 3 zeigt einen Stabilitätstest für eine mit Propanthiol nachbelegte Monoschicht des Oligonukleotids 1. Zur Zeit t = 0 liegt der mit Propanthiol nachbelegte Oligonukleotidmonolayer in 500 m mol/l P-Puffer bei pH 7 vor. Bei t = 1h wurde ein Pufferwechsel zu 500 mmol/l P-Puffer mit pH 9 vorgenommen. Der Resonanzwinkel ändert sich hierbei aufgrund des höheren Brechungsindex des Puffers. Nachfolgend wurde bei t = 2 h zu dem ursprünglichen 500 mmol/l P-Puffer, pH 7 gewechselt, wobei der Resonanzwinkel auf seinen Ursprungswert zurückgeht. Die Stoffmenge auf der Oberfläche hat sich dementsprechend durch die Behandlung bei pH 9 nicht verändert. Bei t = 5 h wurde der Puffer durch 2 mol/l NaOH ersetzt und abschließend bei t = 5.5 h wieder zu 500 mmol/l P-Puffer, pH 7 gewechselt. Auch unter diesen Bedingungen bleibt der Monolayer stabil und es tritt kein Materialverlust auf.

### Überprüfung der Hybridisierbarkeit:

Zur Überprüfung der Hybridisierbarkeit wurden die obigen Oligonukleotid-Monoschichten mit einem zweifach 5'-Ferrocenessigsäure-gelabelten Gegenstrang ([FcAc-Y]₂-C GGA TAA CAC AGT CAC CT; Y= Amino Introducing Reagent mit C3-Spacer; Chemgene) hybridisiert. Die Hybridisierung erfolgte durch Inkubation der Monoschicht mit einer auf 95°C temperierten 1 µmol/l Lösung des Gegenstrangs in 100 mmol Natriumsulfat-Lösung und anschließendes Abkühlen über einen Zeitraum von mindestens 2 Stunden.

Zur elektrochemischen Charakterisierung wurden als voltammetrische Methoden Square-Wave-Voltammetrie und zyklische Voltammetrie eingesetzt. Beide Methoden können zur Detektion von oberflächengebundenen Redoxlabeln (hier der Ferrocenessigsäure) eingesetzt werden.

In der Square-Wave-Voltammetrie wird einer linearen Spannungsrampe eine Rechteckspannung mit Frequenz f und Amplitude E^{∼} überlagert (im Bsp. f = 10 Hz, E^{∼} = 20 mV rms) und der Strom erst gegen Ende eines jeden Pulses detektiert. Hierdurch werden kapazitive Ladeströme nahezu eliminiert, was in einem voltammetrischen Peak resultiert. Ein relativer Vergleich der Peakströme ist sehr leicht möglich, wohingegen die absolute Quantifizierung der beteiligten Ladungen nicht trivial ist.

In der zyklischen Voltammetrie werden Dreieck-Spannungsrampen gefahren und der resultierende Strom detektiert. Ausgeprägte kapazitive Ladeströme können hier die Bestimmung der faradayischen Ströme erschweren. Durch Integration der Peakflächen lassen sich allerdings die Anzahl der transferierten Ladungen und somit auch die Anzahl der Redoxlabel bestimmen.

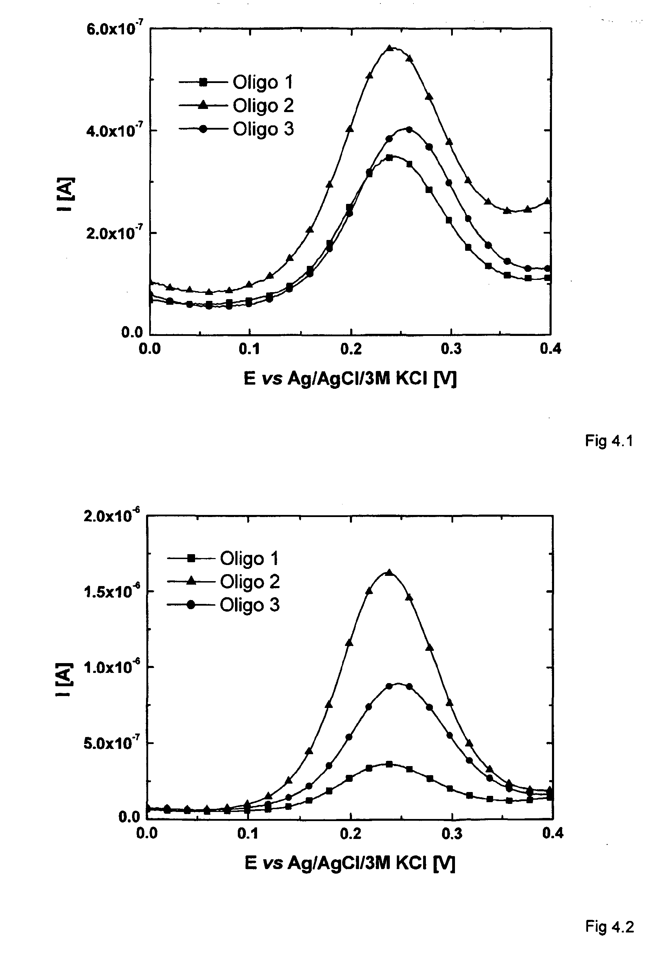

Mittels der oben beschriebenen Square-Wave-Voltammetrie wurde überprüft, ob die Redox-Label des hybridisierten Gegenstrangs detektiert werden können. Die Figuren 4.1 und 4.2 zeigen die Square-Wave-Voltammogramme (f = 10 Hz, E⁻ = 20 mV rms) für die mit Propanthiol (Fig. 4.1) und Hydroxypropanthiol (Fig 4.2) nachbelegten und mit dem redoxmarkierten Gegenstrang hybridisierten Oligonukleotidmonolayer 1-3. Man erkennt bei allen Monolayern einen deutlichen Peak bei +0.23 V (vs Ag/AgCl/3M KCI), welcher von der Oxidation der Ferrocenessigsäure am Gegenstrang herrührt und für die erfolgreiche Hybridisierung der Monolayer spricht. Die Peakströme und somit die Hybridisierungseffizienz unterscheiden sich allerdings in Abhängigkeit von der Nachbelegung und dem verwendeten Oligonukleotid.

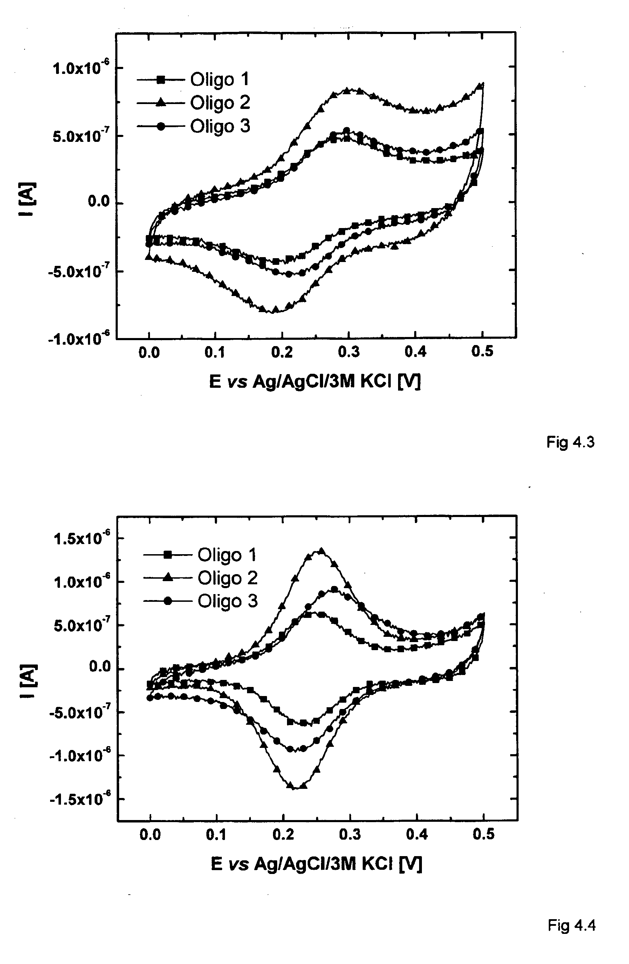

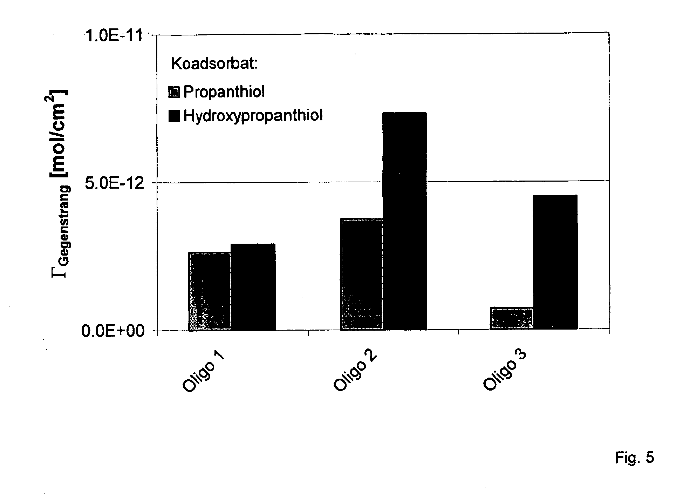

Zur Quantifizierung wurde die zyklische Voltammetrie herangezogen. Durch Integration der zyklischen Voltammogramme (v = 500 mV/s) in Figur 4.3 und 4.4 wurde die Anzahl der Redoxlabel und somit die jeweilige Oberflächenkonzentration Γ des Gegenstrangs (Rauhigkeitsfaktor = 2, Anzahl der Label pro Target = 2) bestimmt und in Figur 5 aufgetragen. Die Oberflächenkonzentration Γ ist ein direktes Maß für die Hybridisierbarkeit der Oligonukleotidmonolayer.

Von den drei Oligonukleotiden des Beispiels 13 zeigt das Oligonukleotid 2 die beste Hybridisierbarkeit. Die Hybridisierbarkeit der Hydroxypropanthiol-nachbelegten ssDNA-Monoschichten ist dabei deutlich größer als die der entsprechenden Propanthiolnachbelegten Monoschichten. Die Oberflächenbelegungen liegen zwischen 1·10⁻¹² - 7·10⁻¹² mol/cm² und somit in einem Bereich der zuvor auch von anderen Gruppen gefunden wurde (Herne T.M., Tarlov M.J. J. Am. Chem. Soc. 1997, 119, 8916-8920)

Alle obigen elektrochemischen Experimente wurden in einem 3-Elektroden-Setup mit der zu untersuchenden Gold-Arbeitselektrode, einer Pt-Gegenelektrode sowie einer Referenzelektrode (Ag/AgCl/3M KCI) an einem Potentiostaten Autolab12 der Firma Ecochemie ((Niederlande) durchgeführt.

### Beispiel 15

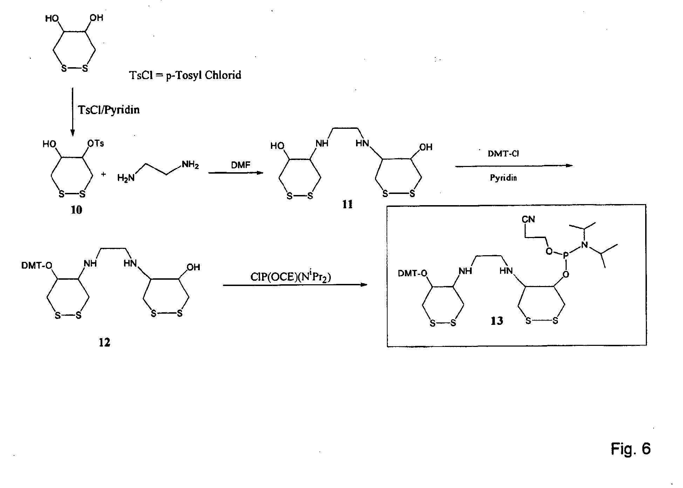

Zu einer Lösung von 2 mmol trans-1,2-Dithian-4,5-diol in wasserfreiem Pyridin werden 2 mmol p-Tosylchlorid gegeben. Nach 2 Stunden Rühren bei Raumtemperatur wird das Lösungsmittel abgedampft. 0.5 eq Ethylendiamin in DMF in Gegenwart von NaH werden 10 min bei Raumtemperatur gerührt. Das Produkt **10** (siehe Fig. 6) wird in DMF gelöst und zu der obigen Ethylendiamin Lösung hinzugefügt. Unter Rückfluss wird die Reaktionsmischung 6 Stunden gerührt. Das gewünschte Produkt **11** (siehe Fig. 6) wird mittels Kieselgel Säulenchromatographie isoliert und mit 1 eq DMT-CI in Pyridin zu Produkt **12** (siehe Fig. 6) umgesetzt. Das DMT-geschützte Produkt wird über Kieselgel mit einer Mischung von Ethylacetat/n-Heptan mit 1 % Et₃N als Laufmittel chromatographiert. Unter Argon-Atmosphäre wird 1 mmol von Produkt **12** (siehe Fig. 6) in 10 ml wasserfreiem DCM gelöst. Die Lösung wird im Eisbad gekühlt und unter Rühren werden 4 eq N,N'-Diisopropylethylamin tropfenweise zugegeben. Dazu werden mit einer Spritze 1.2 eq Chlor-(2-cyanoethoxy)(diisopropylamino)-phosphin getropft. Nach 1.5 Stunden unter Rühren bei Raumtemperatur wird das Reaktionsgemisch mit DCM verdünnt und mit Standardmethoden aufgearbeitet. Zur Reinigung des Produktes **13** (siehe Fig. 6) wird über Kieselgel chromatographiert (Laufmittel: Ethylacetat /n-Heptan in Gegenwart von 1 % Et₃N).

### Beispiel 16

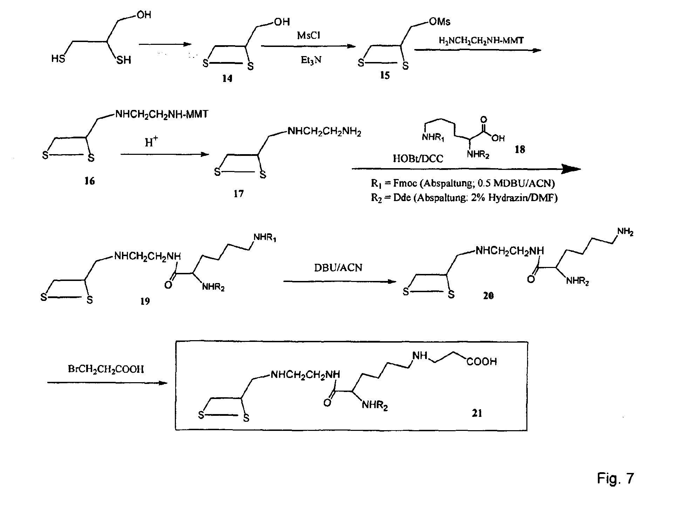

1 g (8 mmol) von 2,3-Dimercapto-1-propanol wird in ACN gelöst und mit Luft (Sauerstoff) oxidiert. Die entsprechende zyklische Verbindung **14** (siehe Fig. 7) wird mit MsCl (Mesylchlorid) in Gegenwart von Et₃N umgesetzt. Nach 2 h Rühren bei Raumtemperatur wird das Lösungsmittel einrotiert. Eine Lösung von 1 eq MMT-Ethylendiamin wird mit NaH in DMF behandelt. Nach 30 min Rühren bei Raumtemperatur wird 1 eq von Verbindung **14** (siehe Fig. 7) in DMF zu der obigen MMT-Ethylendiamin Lösung gegeben. Die Reaktionsmischung wird unter Rückfluss 4 Stunden gerührt. Das Lösungsmittel wird am Rotationsverdampfer eingeengt, der Rückstand mit Standardmethoden aufgearbeitet und mittels Kieselgel-Säulen-Chromatographie aufgereinigt.

Die MMT Gruppe der Verbindung **16** (siehe Fig. 7) wird mit einer säurehaltigen Lösung 2% DCA/DCM abgespalten. 1 eq von Verbindung **17** (siehe Fig. 7) wird in DMF aufgenommen und mit N-α-Fmoc-N-ε-1-(4,4-dimethyl-2,6-dioxocyclohex-1-ylidene)ethyl-L-Lysin (Produkt **18** (siehe Fig. 7)) in Gegenwart von HOBt und DCC umgesetzt. Die Reaktion wird über Nacht bei Raumtemperatur durchgeführt. Das Lösungsmittel wird abgezogen und der Rückstand mit Standardmethoden aufgearbeitet und mittels Kieselgel-Säulenchromatographie mit Ethylacetat/n-Heptan als Laufmittel aufgereinigt.

Die Fmoc Gruppe der Verbindung **19** (siehe Fig. 7) wird selektiv mit einer Lösung von 0.5 M DBU in ACN abgespalten. Die freie Amino Gruppe des Produkts **20** (siehe Fig. 7) wird mit BrCH₂CH₂COOH umgesetzt, um das Produkt **21** (siehe Fig. 7) als Hauptprodukt zu liefern. Das Rohprodukt wird mittels Kieselgel Säulenchromatographie mit Ethylacetat/n-Heptan als Laufmittel aufgereinigt.

### Beispiel 17

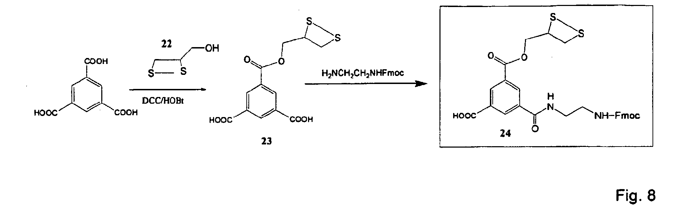

Zu einer Lösung von 1 g (4.7 mmol) 1,3,5-Benzoltricarbonsäure in wasserfreiem DMF werden jeweils 1 eq von Verbindung **22** (siehe Fig. 8), DCC und HOBt gegeben. Die Reaktionsmischung wird über Nacht bei Raumtemperatur gerührt. Das Lösungsmittel wird eingeengt und das gewünschte Produkt **23** (siehe Fig. 8) wird mittels Kieselgel Chromatographie isoliert. Die Verbindung **23** (siehe Fig. 8) wird in DMF aufgenommen und mit Fmoc-Ethylendiamin in Gegenwart von DCC und HOBt eingesetzt. Nach dem Einengen des Lösungsmittels wird das Rohprodukt über Kieselgel mit einer Mischung von Ethylacetat/n-Heptan als Laufmittel chromatographiert.

### Beispiel 18

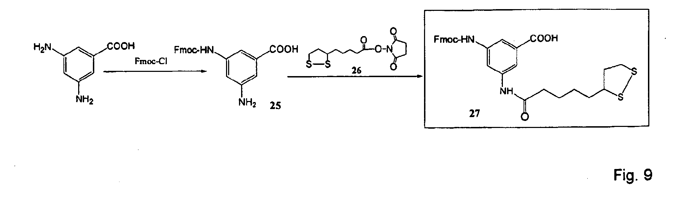

Zu einer Lösung von 2 g (13 mmol) 3,5-Diaminobenzoesäure in 40 ml wasserfreiem Pyridin werden 1.2 eq Fmoc-Cl ((9-Flurorenylmethyl)-chloroformiat) hinzugefügt. Nach 2 Stunden Rühren bei Raumtemperatur wird das Lösungsmittel abgedampft und das Rohprodukt mittels Kieselgel-Säulenchromatographie mit Ethylacetat/n-Heptan als Laufmittel aufgereinigt. Die Verbindung **25** (siehe Fig. 9) wird in einer Mischung ACN/Dioxan gelöst und mit dem N-Hydroxysuccinimid Aktivester der Liponsäure umgesetzt. Die Reaktionsmischung wird über Nacht bei Raumtemperatur gerührt. Das Lösungsmittel wird einrotiert und der Rückstand wird in DCM aufgenommen und mit Standardmethoden aufgearbeitet. Das Rohprodukt wird über Kieselgel mit Ethylacetat/n-Heptan als Laufmittel chromatographiert, um das gewünschte Produkt **27** (siehe Fig. 9) zu isolieren.

## Patentansprüche

1. Verbindungen der Formel 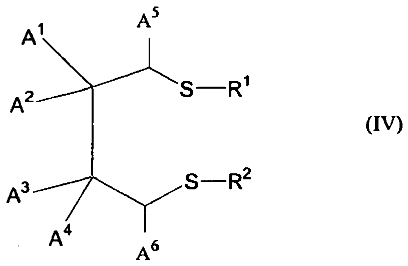
wobei A¹, A³, A⁵ und A⁶ gleich H sind,
A⁴ gleich Y² ist,
A² ein Alkylrest mit 1-22 C-Atomen, ein Heteroalkylrest mit 1-22 C-Atomen, ein Cycloalkylrest mit 1-22 C-Atomen ist und Schutzgruppe-Y¹ umfasst, oder A² gleich Schutzgruppe-Y¹ ist,
R¹ und R² gleiche oder verschiedene H oder Schwefel-Schutzgruppen sind, wobei die beiden S-Atome auch eine Disulfid-Brücke ausbilden können und in diesem Fall R¹, R² nicht vorhanden sind,
Schutzgruppe-Y¹ gleich Schutzgruppe-S, Schutzgruppe-NH, Schutzgruppe-NR⁴, Schutzgruppe-O, Schutzgruppe-S-S, Schutzgruppe-OOC ist, Schutzgruppe gleich 9-Fluorenylmethoxycarbonyl oder Dimethoxytrityl ist,
Y² gleich -OH, -NH₂, -NHR³, -NR³R⁴, -COOH, -COCI, -COOCO-R⁶, -CONH₂, -CONHR³, -COOR³, -SO₃H, -SO₃Cl, -SH, -S-SR³, -CHO, -COR³, -C₂H₃O, Halogen, -N₃, -NH-NH₂, -NCO, -NCS ist
wobei R³ Alkyl, Heteroalkyl, Aryl, Cycloalkyl oder eine Schutzgruppe ist,
R⁴ eine Schutzgruppe ist, wobei R⁴ und R³ gleich oder verschieden sein können,
R⁵Alkyl, Aryl, Cycloalkyl ist, und
R⁶ Alkyl, Heteroalkyl, Aryl oder Cycloalkyl ist,
wobei Y² auch eine Gruppe der Formel (II) oder (III) sein kann, 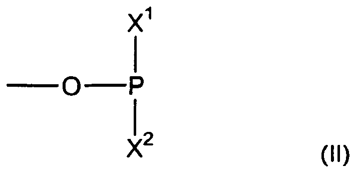 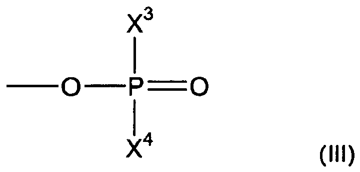
wobei
X¹ ein Halogen oder ein substituiertes Amin ist,
X² ein Alkyl-, Alkoxy-, Aryloxy-Rest oder ein Cyanoderivat eines Alkyl-, Alkoxy-, Aryloxy-Restes ist,
X³ ein Halogen, eine Aminofunktion oder Sauerstoff ist und
X⁴ ein Alkyl-, Alkoxy-, Aryloxy-Rest ist oder X⁴ gleich H ist für den Fall, dass X³ = Sauerstoff ist.

2. Verbindungen der Formel 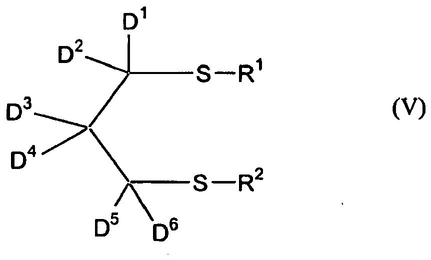
wobei D¹, D², D³ und D⁵ gleich H sind,
D⁶ gleich Y² ist,
D⁴ ein Alkylrest mit 1-22 C-Atomen, ein Heteroalkylrest mit 1-22 C-Atomen, ein Cycloalkylrest mit 1-22 C-Atomen ist und Schutzgruppe-Y¹ umfasst, oder D⁴ gleich Schutzgruppe-Y¹ ist,
R¹ und R² gleiche oder verschiedene H oder Schwefel-Schutzgruppen sind, wobei die beiden S-Atome auch eine Disulfid-Brücke ausbilden können und in diesem Fall R¹, R² nicht vorhanden sind,
Schutzgruppe-Y¹ gleich Schutzgruppe-S, Schutzgruppe-NH, Schutzgruppe-NR⁴, Schutzgruppe-O, Schutzgruppe-S-S, Schutzgruppe-OOC ist, Schutzgruppe gleich 9-Fluorenylmethoxycarbonyl oder Dimethoxytrityl ist,
Y² gleich -OH, -NH₂, -NHR³, -NR³R⁴, -COOH, -COCI, -COOCO-R⁶, -CONH₂, -CONHR³, -COOR³, -SO₃H, -SO₃Cl, -SH, -S-SR³, -CHO, -COR³, -C₂H₃O, Halogen, -N₃, -NH-NH₂, -NCO, -NCS ist
wobei R³Alkyl, Heteroalkyl, Aryl, Cycloalkyl oder eine Schutzgruppe ist,
R⁴ eine Schutzgruppe ist, wobei R⁴ und R³ gleich oder verschieden sein können,
R⁵Alkyl, Aryl, Cycloalkyl ist, und
R⁶ Alkyl, Heteroalkyl, Aryl oder Cycloalkyl ist,
wobei Y² auch eine Gruppe der Formel (II) oder (III) sein kann, 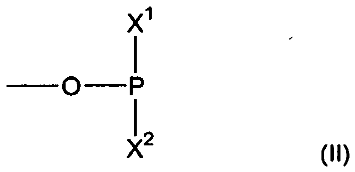 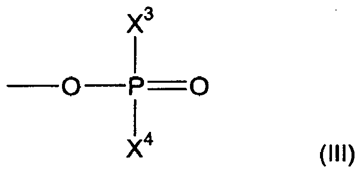
wobei
X¹ ein Halogen oder ein substituiertes Amin ist,
X² ein Alkyl-, Alkoxy-, Aryloxy-Rest oder ein Cyanoderivat eines Alkyl-, Alkoxy-, Aryloxy-Restes ist,
X³ ein Halogen, eine Aminofunktion oder Sauerstoff ist und
X⁴ ein Alkyl-, Alkoxy-, Aryloxy-Rest ist oder X⁴ gleich H ist für den Fall, dass X³ = Sauerstoff ist.

3. Verbindungen der Formel 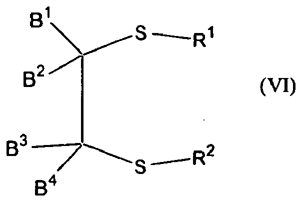
wobei B¹, B² und B³ gleich H sind,
B⁴ ein Alkylrest mit 1-22 C-Atomen, ein Heteroalkylrest mit 1-22 C-Atomen, ein Cycloalkylrest mit 1-22 C-Atomen ist und die Gruppen Schutzgruppe-Y¹ und Y² umfasst,
R¹ und R² gleiche oder verschiedene H oder Schwefel-Schutzgruppen sind, wobei die beiden S-Atome auch eine Disulfid-Brücke ausbilden können und in diesem Fall R¹, R² nicht vorhanden sind,
Schutzgruppe-Y¹ gleich Schutzgruppe-S, Schutzgruppe-NH, Schutzgruppe-NR⁴, Schutzgruppe-O, Schutzgruppe-S-S, Schutzgruppe-OOC ist, Schutzgruppe gleich 9-Fluorenylmethoxycarbonyl oder Dimethoxytrityl ist,
Y² gleich -OH, -NH₂, -NHR³, -NR³R⁴, -COOH, -COCI, -COOCO-R⁶, -CONH₂, -CONHR³, -COOR³, -SO₃H, -SO₃Cl, -SH, -S-SR³, -CHO, -COR³, -C₂H₃O, Halogen, -N₃, -NH-NH₂, -NCO, -NCS ist
wobei R³ Alkyl, Heteroalkyl, Aryl, Cycloalkyl oder eine Schutzgruppe ist,
R⁴ eine Schutzgruppe ist, wobei R⁴ und R³ gleich oder verschieden sein können,
R⁵ Alkyl, Aryl, Cycloalkyl ist, und
R⁶ Alkyl, Heteroalkyl, Aryl oder Cycloalkyl ist,
wobei Y² auch eine Gruppe der Formel (II) oder (III) sein kann, 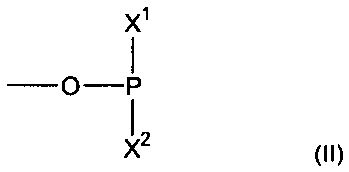 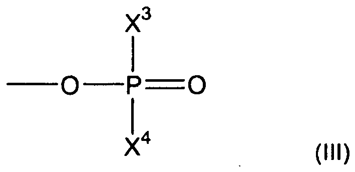 wobei
X¹ ein Halogen oder ein substituiertes Amin ist,
X² ein Alkyl-, Alkoxy-, Aryloxy-Rest oder ein Cyanoderivat eines Alkyl-, Alkoxy-, Aryloxy-Restes ist,
X³ ein Halogen, eine Aminofunktion oder Sauerstoff ist und
X⁴ ein Alkyl-, Alkoxy-, Aryloxy-Rest ist oder X⁴ gleich H ist für den Fall, dass X³ = Sauerstoff ist.

4. Verbindungen nach einem der vorhergehenden Ansprüche, worin Y² der Formel (II) oder (III) entspricht, wobei
X¹ ein Halogen und X² Methyl oder R⁷O- ist,
oder X² gleich R⁷O- und X¹ gleich -NR⁸R⁹ ist, wobei R⁷ ein Alkyl-, ein Cycloalkyl-, ein Aryl-, ein Cyanoalkyl-, ein Cyanocycloalkyl- oder ein Cyanoaryl-Rest ist und wobei R⁸ und R⁹ unabhängig voneinander Alkyl-, Heteroalkyl-, Cycloalkyl-, Aryl-Reste sind oder R⁸ und R⁹ miteinander verbunden sind, sodass sie mit dem N-Atom eine zyklische Struktur mit 4 bis 7 C-Atomen bilden, wobei ein C-Atom der zyklischen Struktur durch O oder S ersetzt sein kann,
oder X³ = O⁻ und X⁴ = H oder R¹⁰O- ist, wobei R¹⁰ eine Schutzgruppe darstellt.

5. Verbindungen nach Anspruch 4, wobei R⁷ eine basenlabile Schutzgruppe, insbesondere eine basenlabile Schutzgruppe ausgewählt aus ß-Cyanoethyl, β-Nitroethyl, 2,2,2-Trichlorethyl, Methyl, 1,1-Dimethyl-2,2,2-Thrichlorethyl, 2,2,2-Tribromethyl, Benzyl, o-Chlorphenyl, p-Nitrophenylethyl, 2-Methylsulfonylethyl und 1,1-Dimethyl-2-cyanoethyl ist.

6. Verbindungen nach einem der Ansprüche 4 und 5, wobei R⁸ und R⁹ unabhängig voneinander Alkyl-Reste bestehend aus 1-16 C-Atomen, insbesondere Alkyl-Reste bestehend aus 1-6 C-Atomen, Cycloalkyl-Reste bestehend aus 3-8 C-Atomen, Aryl-Reste bestehend aus 6-20 C-Atomen sind oder R⁸ und R⁹ miteinander verbunden sind, sodass sie mit dem N-Atom eine zyklische Struktur mit 4 bis 7 C-Atomen bilden, wobei ein C-Atom der zyklischen Struktur durch O oder S ersetzt sein kann.

7. Verbindungen nach Anspruch 6, wobei R⁸ und R⁹ Isopropyl, Butyl, Hexyl, Nonyl, Dodecyl, Hexadecyl, Cyclopropyl, Cyclobutyl, Cyclohexyl, Cyclooctyl, Phenyl, Tolyl, Benzyl, Xylyl, Naphthyl, Morpholino, Piperidinyl oder Thiomorpholino sind.

8. Verbindungen nach einem der vorhergehenden Ansprüche, wobei R¹ und R² gleich Trityl, 4,4'-Dimethoxytrityl, 4-Monomethoxytrityl, 9-Fluorenylmethyl, 9-Fluorenylmtehoxycarbonyl, 2,4-Dinitrophenylethyl, 2,4,6-Trimethoxybenzyl, 4-Methoxybenzyl oder Allyloxycarbonylaminomethyl sind.

9. Verwendung der Verbindungen nach den vorhergehenden Ansprüchen zur Modifikation von Oligomeren.

10. Verwendung der Verbindungen nach den Ansprüchen 1 bis 8 zur Immobilisierung von modifizierten Oligomeren auf Oberflächen.

11. Verwendung der Verbindungen nach den Ansprüchen 1 bis 8 zur Anbindung von enzymatischen, chromogenen, fluorogenen, radioaktiven oder chemiluminiscenten Labeln, von in Nukleinsäureoligomere interkalierenden Substanzen, von Metallen, von Metallionen, Hormonen, Proteinen, Peptiden, nukleolytischen und proteolytischen Agentien, Biotin, Antigenen, Haptenen, Antikörpern oder Rezeptoren an Moleküle oder Oligomere.

12. Verwendung der Verbindungen nach den Ansprüchen 1 bis 8 bei der automatischen Synthese von Oligomeren.

13. Verwendung nach einem der Ansprüche 9 bis 12, wobei es sich bei den Oligomeren um Oligonukleotide, Polypeptide, PNA oder LNA (Locked Nucleic Acid) handelt.

## Claims

1. Compounds of formula 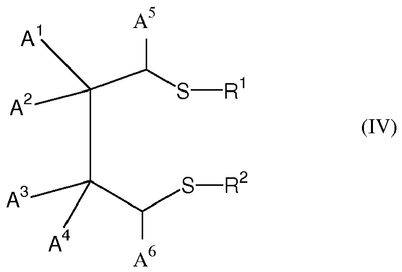
wherein A¹, A³, A⁵ and A⁶ are H,
A⁴ is Y²,
A² is an alkyl group with 1-22 C atoms, a heteroalkyl group with 1-22 C atoms, or a cycloalkyl group with 1-22 C atoms and comprises protecting group Y¹, or A² is the protecting group Y¹,
R¹ and R² are identical or different H or sulphur protecting groups, wherein the two S atoms may also form a disulphide bridge and in said case R¹, R² are not present,
protecting group Y¹ is protecting group S, protecting group NH, protecting group NR⁴, protecting group O, protecting group S-S or protecting group OOC,
protecting group is 9-fluorenylmethoxycarbonyl or dimethoxytrityl,
Y² is -OH, -NH₂, -NHR³, -NR³R⁴, -COOH, -COCl, -COOCO-R⁶, -CONH₂,-CONHR³, -COOR³, -SO₃H, -SO₃Cl, -SH, -S-SR³, -CHO, -COR³, -C₂H₃O, halogen,-N₃, -NH-NH₂, -NCO or -NCS,
wherein R³ is alkyl, heteroalkyl, aryl, cycloalkyl or a protecting group,
R⁴ is a protecting group, wherein R⁴ and R³ can be the same or different,
R⁵ is alkyl, aryl or cycloalkyl, and
R⁶ is alkyl, heteroalkyl, aryl or cycloalkyl,
wherein Y² may also be a group of formula (II) or (III), 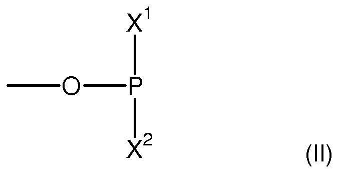 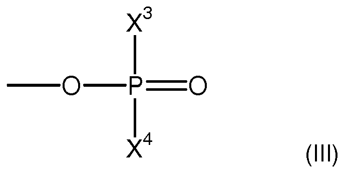 wherein
X¹ is a halogen or a substituted amine,
X² is an alkyl group, alkoxy group, aryloxy group or a cyano derivative of an alkyl group, alkoxy group or aryloxy group,
X³ is a halogen, an amino function or oxygen, and
X⁴ is an alkyl group, alkoxy group or aryloxy group, or X⁴ is H in case X³ = oxygen.

2. Compounds of formula 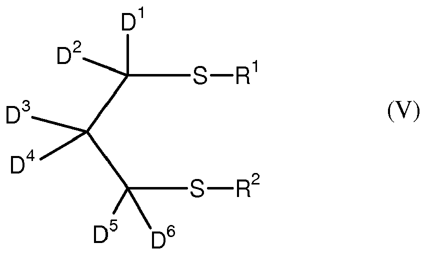
wherein D¹, D², D³ and D⁵ are H,
D⁶ is Y²,
D⁴ is an alkyl group with 1-22 C atoms, a heteroalkyl group with 1-22 C atoms, or a cycloalkyl group with 1-22 C atoms and comprises protecting group Y¹, or D⁴ is protecting group Y¹,
R¹ and R² are identical or different H or sulphur protecting groups, wherein the two S atoms may also form a disulphide bridge and in said case R¹, R² are not present,
protecting group Y¹ is protecting group S, protecting group NH, protecting group NR⁴, protecting group O, protecting group S-S, protecting group OOC,
protecting group is 9-fluorenylmethoxycarbonyl or dimethoxytrityl,
Y² is -OH, -NH₂, -NHR³, -NR³R⁴, -COOH, -COCl, -COOCO-R⁶, -CONH₂, -CONHR³,-COOR³, -SO₃H, -SO₃Cl, -SH, -S-SR³, -CHO, -COR³, -C₂H₃O, halogen, -N₃, -NH-NH₂, -NCO or -NCS,
wherein R³ is alkyl, heteroalkyl, aryl, cycloalkyl or a protecting group,
R⁴ is a protecting group, wherein R⁴ and R³ can be the same or different,
R⁵ is alkyl, aryl or cycloalkyl, and
R⁶ is alkyl, heteroalkyl, aryl or cycloalkyl,
wherein Y² may also be a group of formula (II) or (III), 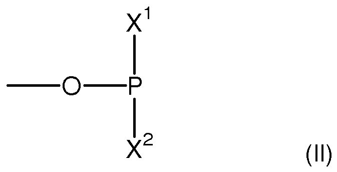 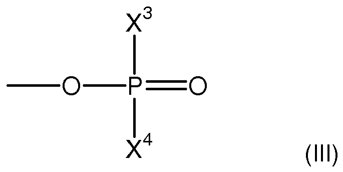 wherein
X¹ is a halogen or a substituted amine,
X² is an alkyl group, alkoxy group, aryloxy group or a cyano derivative of an alkyl group, alkoxy group or aryloxy group,
X³ is a halogen, an amino function or oxygen, and
X⁴ is an alkyl group, alkoxy group or aryloxy group, or X⁴ is H in case X³ = oxygen.

3. Compounds of formula 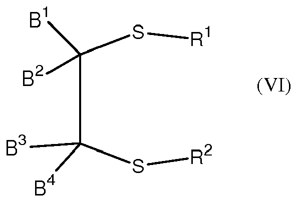
wherein B¹, B² and B³ are H,
B⁴ is an alkyl group with 1-22 C atoms, a heteroalkyl group with 1-22 C atoms, or a cycloalkyl group with 1-22 C atoms and comprises the groups protecting group Y¹ and Y²,
R¹ and R² are identical or different H or sulphur protecting groups, wherein the two S atoms may also form a disulphide bridge and in said case R¹, R² are not present,
protecting group Y¹ is protecting group S, protecting group NH, protecting group NR⁴, protecting group O, protecting group S-S, protecting group OOC,
the protecting group is 9-fluorenylmethoxycarbonyl or dimethoxytrityl,
Y² is -OH, -NH₂, -NHR³, -NR³R⁴, -COOH, -COCl, -COOCO-R⁶, -CONH₂,-CONHR³, -COOR³, -SO₃H, -SO₃Cl, -SH, -S-SR³, -CHO, -COR³, -C₂H₃O, halogen,-N₃, -NH-NH₂, -NCO or -NCS,
wherein R³ is alkyl, heteroalkyl, aryl, cycloalkyl or a protecting group,
R⁴ is a protecting group, wherein R⁴ and R³ can be the same or different,
R⁵ is alkyl, aryl or cycloalkyl, and
R⁶ is alkyl, heteroalkyl, aryl or cycloalkyl,
wherein Y² may also be a group of formula (II) or (III), 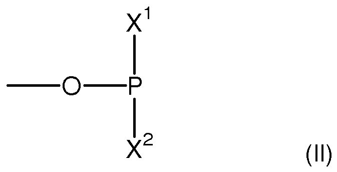 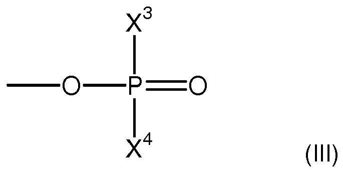 wherein
X¹ is a halogen or a substituted amine,
X² is an alkyl group, alkoxy group, aryloxy group or a cyano derivative of an alkyl group, alkoxy group or aryloxy group,
X³ is a halogen, an amino function or oxygen, and
X⁴ is an alkyl group, alkoxy group or aryloxy group, or X⁴ is H in case X³ = oxygen.

4. Compounds according to one of the preceding claims, wherein Y² corresponds to formula (II) or (III), wherein
X¹ is a halogen and X² is methyl or R⁷O-,
or X² is R⁷O- and X¹ is -NR⁸R⁹, wherein R⁷ is an alkyl group, a cycloalkyl group, an aryl group, a cyanoalkyl group, a cyanocycloalkyl group, or a cyanoaryl group, and wherein R⁸ and R⁹ independently of one another are alkyl groups, heteroalkyl groups, cycloalkyl groups or aryl groups, or R⁸ and R⁹ are interconnected, such that they form, together with the N atom, a cyclic structure with 4 to 7 C atoms, wherein one C atom of the cyclic structure can be replaced by O or S,
or X³ = O and X⁴ = H or R¹⁰ is O-, wherein R¹⁰ represents a protecting group.

5. Compounds according to claim 4, wherein R⁷ is a base-labile protecting group, in particular a base-labile protecting group selected from β-cyanoethyl, β-nitroethyl, 2,2,2-trichloroethyl, methyl, 1,1-dimethyl-2,2,2-trichloroethyl, 2,2,2-tribromethyl, benzyl, o-chlorophenyl, p-nitrophenylethyl, 2-methylsulfonylethyl and 1,1-dimethyl-2-cyanoethyl.

6. Compounds according to one of claims 4 and 5, wherein R⁸ and R⁹ independently of one another are alkyl groups consisting of 1-16 C atoms, in particular alkyl groups consisting of 1-6 C atoms, cycloalkyl groups consisting of 3-8 C atoms, aryl groups consisting of 6-20 C atoms, or R⁸ and R⁹ are interconnected, such that they form, together with the N atom, a cyclic structure with 4 to 7 C atoms, wherein one C atom of the cyclic structure can be replaced by O or S.

7. Compounds according to claim 6, wherein R⁸ and R⁹ are isopropyl, butyl, hexyl, nonyl, dodecyl, hexadecyl, cyclopropyl, cyclobutyl, cyclohexyl, cyclooctyl, phenyl, tolyl, benzyl, xylyl, naphthyl, morpholino, piperidinyl or thiomorpholino.

8. Compounds according to one of the preceding claims, wherein R¹ and R² are trityl, 4,4'-dimethoxytrityl, 4-monomethoxytrityl, 9-fluorenylmethyl, 9-fluorenylmethoxycarbonyl, 2,4-dinitrophenylethyl, 2,4,6-trimethoxybenzyl, 4-methoxybenzyl or allyloxycarbonylaminomethyl.

9. Use of the compounds according to one of the preceding claims for modifying oligomers.

10. Use of the compounds according to claims 1 to 8 for immobilising modified oligomers on surfaces.

11. Use of the compounds according to claims 1 to 8 for binding enzymatic, chromogenic, fluorogenic, radioactive or chemiluminescent labels, of substances intercalating in nucleic acid oligomers, of metals, of metal ions, hormones, proteins, peptides, nucleolytic and proteolytic agents, biotin, antigens, haptens, antibodies or receptors, to molecules or oligomers.

12. Use of the compounds according to claims 1 to 8 in the automatic synthesis of oligomers.

13. Use according to one of claims 9 to 12, wherein the oligomers are oligonucleotides, polypeptides, PNA or LNA (locked nucleic acid).

## Revendications

1. Composés répondant à la formule 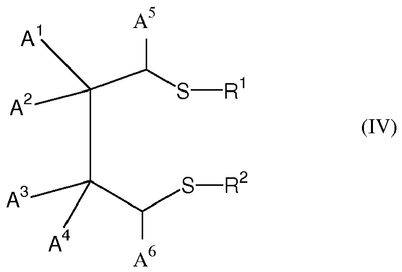
A¹, A³, A⁵ et A⁶ représentant H,
A⁴ représentant Y²,
A² représentant un groupe alkyle dont le nombre d'atomes de C est compris entre 1 à 22, un groupe hétéroalkyle dont le nombre d'atomes de C est compris entre 1 à 22, un groupe cycloalkyle dont le nombre d'atomes de C est compris entre 1 à 22, et comprenant un groupe protecteur-Y¹, ou A² représentant un groupe protecteur-Y¹,
R¹ et R² étant identiques ou différents et représentant chacun H ou groupes protecteures soufrés, les deux atomes de S pouvant également former un pont disulfure, R¹, R² n'étant pas présents dans ce dernier cas,
groupe protecteur-Y¹ représentant groupe protecteur-S, groupe protecteur-NH, groupe protecteur-NR⁴, groupe protecteur-O, groupe protecteur-S-S, groupe protecteur-OOC,
groupe protecteur représentant 9-fluorénylméthoxycarbonyle ou diméthoxytrityle, Y² représentant -OH, -NH₂, -NHR³, -NR³R⁴, -COOH, -COCI, -COOCO-R⁶, -CONH₂, -CONHR³, -COOR³, -SO₃H, -SO₃Cl, -SH, -S-SR³, -CHO, -COR³, -C₂H₃O, halogène, -N₃, -NH-NH₂, -NCO, -NCS,
R³ représentant alkyle, hétéroalkyle, aryle, cycloalkyle ou un groupe protecteur,
R⁴ représentant un groupe protecteur, R⁴ et R³ pouvant être identiques ou différents,
R⁵ représentant alkyle, aryle, cycloalkyle, et
R⁶ représentant alkyle, hétéroalkyle, aryle ou cycloalkyle,
Y² pouvant également représenter un groupe répondant à la formule (II) ou (III), 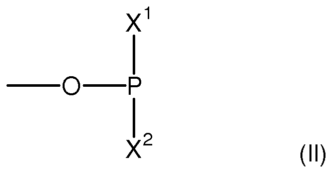 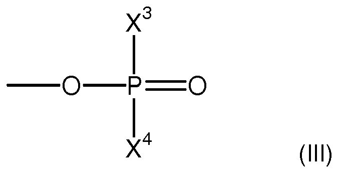 dans laquelle
X¹ représente un halogène ou une amine substituée,
X² représente un groupe alkyle, alkoxy, aryloxy ou bien un dérivé cyano d'un groupe alkyle, alkoxy, aryloxy,
X³ représente un halogène, une fonction amine ou un oxygène, et
X⁴ représente un groupe alkyle, alkoxy, aryloxy ou bien, si X³ = oxygène, X⁴ représente H.

2. Composés répondant à la formule 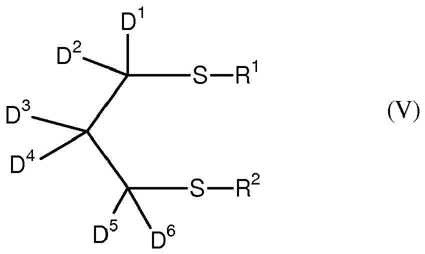
D¹, D¹, D³ et D⁵ représentant H,
D⁶ représentant Y²,
D⁴ représentant un groupe alkyle dont le nombre d'atomes de C est compris entre 1 à 22, un groupe hétéroalkyle dont le nombre d'atomes de C est compris entre 1 à 22, un groupe cycloalkyle dont le nombre d'atomes de C est compris entre 1 à 22, et comprenant un groupe protecteur-Y¹, ou D⁴ représentant un groupe protecteur-Y¹,
R¹ et R² étant identiques ou différents et représentant chacun H ou groupes protecteures soufrés, les deux atomes de S pouvant également former un pont disulfure, R¹, R² n'étant pas présents dans ce dernier cas,
groupe protecteur-Y¹ représentant groupe protecteur-S, groupe protecteur-NH, groupe protecteur-NR⁴, groupe protecteur-O, groupe protecteur-S-S, groupe protecteur-OOC,
groupe protecteur représentant 9-fluorénylméthoxycarbonyle ou diméthoxytrityle,
Y² représentant -OH, -NH₂, -NHR³, -NR³R⁴, -COOH, -COCI, -COOCO-R⁶, -CONH₂, -CONHR³, -COOR³, -SO₃H, -SO₃Cl, -SH, -S-SR³, -CHO, -COR³, -C₂H₃O, halogène, -N₃, -NH-NH₂, -NCO, -NCS,
R³ représentant alkyle, hétéroalkyle, aryle, cycloalkyle ou un groupe protecteur,
R⁴ représentant un groupe protecteur, R⁴ et R³ pouvant être identiques ou différents,
R⁵ représentant alkyle, aryle, cycloalkyle, et
R⁶ représentant alkyle, hétéroalkyle, aryle ou cycloalkyle,
Y² pouvant également représenter un groupe répondant à la formule (II) ou (III), 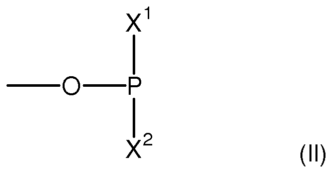 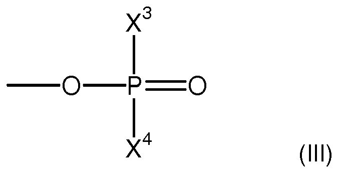 dans laquelle
X¹ représente un halogène ou une amine substituée,
X² représente un groupe alkyle, alkoxy, aryloxy ou bien un dérivé cyano d'un groupe alkyle, alkoxy, aryloxy,
X³ représente un halogène une fonction amine ou un oxygène, et
X⁴ représente un groupe alkyle, alkoxy, aryloxy ou bien, si X³ = oxygène, X⁴ représente H.

3. Composés répondant à la formule 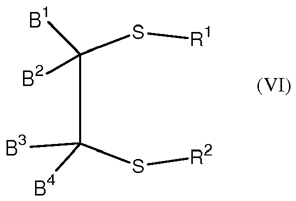
B¹, B² et B³ représentant H,
B⁴ représentant un groupe alkyle dont le nombre d'atomes de C est compris entre 1 à 22, un groupe hétéroalkyle dont le nombre d'atomes de C est compris entre 1 à 22, un groupe cycloalkyle dont le nombre d'atomes de C est compris entre 1 à 22 et comprenant les groupes groupe protecteur-Y¹ et Y²,
R¹ et R² étant identiques ou différents et représentant chacun H ou groupes protecteures soufrés, les deux atomes de S pouvant également former un pont disulfure, R¹, R² n'étant pas présents dans ce dernier cas,
groupe protecteur-Y¹ représentant un groupe protecteur-S, groupe protecteur-NH, groupe protecteur-NR⁴, groupe protecteur-O, groupe protecteur-S-S, groupe protecteur-OOC,
groupe protecteur représentant 9-fluorénylméthoxycarbonyle ou diméthoxytrityle,
Y² représentant -OH, -NH₂, -NHR³, -NR³R⁴, -COOH, -COCI, -COOCO-R⁶, -CONH₂, -CONHR³, -COOR³, -SO₃H, -SO₃Cl, -SH, -S-SR³, -CHO, -COR³, -C₂H₃O, halogène, -N₃, -NH-NH₂, -NCO, -NCS,
R³ représentant alkyle, hétéroalkyle, aryle, cycloalkyle ou un groupe protecteur,
R⁴ représentant un groupe protecteur, R⁴ et R³ pouvant être identiques ou différents,
R⁵ représentant alkyle, aryle, cycloalkyle, et
R⁶ représentant alkyle, hétéroalkyle, aryle ou cycloalkyle,
Y² pouvant également représenter un groupe répondant à la formule (II) ou (III), 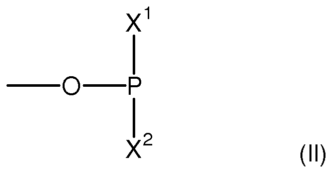 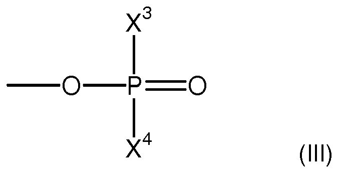 dans laquelle
X¹ représente un halogène ou une amine substituée,
X² représente un groupe alkyle, alkoxy, aryloxy ou bien un dérivé cyano d'un groupe alkyle, alkoxy, aryloxy,
X³ représentant un halogène, une fonction amine ou un oxygène, et
X⁴ représente un groupe alkyle, alkoxy, aryloxy ou bien, si X³ = oxygène, X⁴ représente H.

4. Composés selon l'une des revendications précédentes, Y² répondant à la formule (II) ou (III),
X¹ représentant un halogène et X² représentant méthyle ou R⁷O-,
ou X² représentant R⁷O- et X¹ représentant -NR⁸R⁹, R⁷ représentant un groupe alkyle, cycloalkyle, aryle, cyanoalkyle, cyanocycloalkyle ou cyanoaryle, et R⁸ et R⁹ représentant chacun, indépendamment l'un de l'autre, un groupe alkyle, hétéroalkyle, cycloalkyle, aryle, ou bien R⁸ et R⁹ étant reliés l'un à l'autre pour ainsi former avec l'atome de N une structure cyclique renfermant 4 à 7 atomes de C, l'un des atomes de C de ladite structure cyclique pouvant être remplacé par O ou S,
ou bien X³ = O- et X⁴ = H ou R¹⁰O-, R¹⁰ représentant un groupe protecteur.

5. Composés selon la revendication 4, R⁷ représentant un groupe protecteur labile en présence de bases, notamment un groupe protecteur labile en présence de bases choisi parmi le β-cyanoéthyle, le β-nitroéthyle, le 2,2,2-trichloroéthyle, le méthyle, le 1,1-diméthyl-2,2,2-trichloroéthyle, 2,2,2-tribromoéthyle, le benzyle, le o-chlorophényle, le p-nitrophényléthyle, le 2-méthylsulfonyléthyle et le 1,1-diméthyl-2-cyanoéthyle.

6. Composés selon l'une des revendications 4 et 5, R⁸ et R⁹ représentant, indépendamment l'un de l'autre, des groupes alkyle constitués de 1 à 16 atomes de C, notamment des groupes alkyle constitués de 1 à 6 atomes de C, des groupes cycloalkyle constitués de 3 à 8 atomes de C, des groupes aryles constitués de 6 à 20 atomes de C, ou bien R⁸ et R⁹ étant reliés l'un à l'autre pour ainsi former avec l'atome de N une structure cyclique renfermant 4 à 7 atomes de C, l'un des atomes de C de ladite structure cyclique pouvant être remplacé par O ou S.

7. Composés selon la revendication 6, R⁸ et R⁹ représentant isopropyle, butyle, hexyle, nonyle, dodécyle, hexadécyle, cyclopropyle, cyclobutyle, cyclohexyle, cyclooctyle, phényle, tolyle, benzyle, xylyle, naphtyle, morpholino, pipéridinyle ou thiomorpholino.

8. Composés selon l'une des revendications précédentes, R¹ et R² représentant trityle, 4,4'-diméthoxytrityle, 4-monométhoxytrityle, 9-fluorénylméthyle, 9-fluorénylméthoxycarbonyle, 2,4-dinitrophényléthyle, 2,4,6-triméthoxybenzyle, 4-méthoxybenzyle ou allyloxycarbonylaminométhyle.

9. Utilisation des composés selon les revendications précédentes pour modifier des oligomères.

10. Utilisation des composés selon les revendications 1 à 8 pour immobiliser des oligomères modifiés sur des surfaces.

11. Utilisation des composés selon les revendications 1 à 8 pour relier des marqueurs enzymatiques, chromogènes, fluorogènes, radioactives ou chimiluminescents, des substances s'intercalant dans des oligomères d'acide nucléique, des métaux, des ions métalliques, des hormones, des protéines, des peptides, des agents nucléolytiques et protéolytiques, de la biotine, des antigènes, des haptènes, des anticorps ou des récepteurs à des molécules ou oligomères.

12. Utilisation des composés selon les revendications 1 à 8 dans la synthèse automatique d'oligomères.

13. Utilisation selon l'une des revendications 9 à 12, lesdits oligomères étant des oligonucléotides, des polypeptides, du PNA ou du LNA (Locked Nucleic Acid).
